# EUROPEAN PATENT SPECIFICATION

(11) **EP 0 181 929 B1**
(45) Date of publication and mention of the grant of the patent: **10.08.1994**
(21) Application number: 85902885.4
(22) Date of filing: 24.05.1985
(51) Int. Cl.: C12N 15/85, C12P 19/34

(54) **TRANS-ACTING TRANSCRIPTIONAL FACTORS**
TRANSKRIPTIONSFAKTOREN MIT TRANSAKTIVITÄT
FACTEURS DE TRANSCRIPTION PAR TRANS-ACTIVATION

(30) Priority: 25.05.1984 US 614297
(43) Date of publication of application: 28.05.1986
(73) Proprietor: DANA FARBER CANCER INSTITUTE, Boston Massachusetts 02115 (US)
(72) Inventor: HASELTINE, William, A., Cambridge, MA 02140 (US); SODROSKI, Joseph, G., Cambridge, MA 02155 (US); ROSEN, Craig, A., Brookline, MA 02146 (US)
(74) Representative: Bass, John Henton
(86) International application number: PCT/US85/00985
(87) International publication number: WO 85/05636

(56) References cited:
- US-A- 4 405 712
- PROC. NATL. ACAD. SCI. USA, vol. 81, February 1984, pages 1193-1197; R.B.GAYNOR et al.: "Adenovirus early region 1A protein activates transcription of anonviral gene introduced into mammalian cells by infection or transfection"
- SCIENCE, vol. 225, 27th July 1984, pages 381-385; J.G. SODROSKI et al.: "Trans-acting transcriptional activation of the long terminal repeat of human Tlymphotropic viruses in infected cells"
- Science 225, 27 July 1984, SODROSKI et al."Sequence of the envelope glycoprotein gene of type II humanT lymphopic virus "p.421-424
- Science 225, 27 July 1984, HAELTINE et al; "structure of 3? terminal region of type II human T lymphotropic virus: evidence for new coding region "p 419-421
- Nucleic Acids Research 11(17), September 1983,Tsimanis et al; "the structure of cloned 3? terminal rna region of bovine leukemia virus( BLV )" p.6079-6087
- Nature, 309, 17 May 1984, CHEN et al;" long terminal repeats of human T-dell leukemia virus II genome dertermine target cell specificity " p. 276-279
- Proceedings of the National Academy of Sciences, USA 81, August 1984, SAGATA et al; "bovine leukemia virus: unique structural features of its long terminal repeats and its evolutionary relationship to human t-cell leukemia virus" p. 4741-4745
- Proceedings of the National Academy of Sciences, 81, August 1984, SODROSKI et al; " repetitive structure in the long terminal repeat element of a type II human t-cell leukemia virus" p. 4617-4621

## Description

### FIELD OF THE INVENTION

The present invention relates to the production of genetic products in large amounts, using vector systems which contain certain viral transcriptional control elements which cause trans-acting transcriptional activation of the gene of interest, resulting in greatly multiplied expression of the desired gene product. More particularly, the present invention is directed to the use of trans-acting factors from trans-activating retroviruses to provide augmented expression of desired heterologous gene products, and to methods and products useful in such expression. The present invention utilizes transcriptional control elements from trans-activating retroviruses as transcriptional promoters for heterologous genes on both integrated and unintegrated DNA. The use of the transcriptional control factors of the present invention dramatically increases the rate of transcription of the heterologous gene, and hence the expression of the desired gene product.

### BACKGROUND OF THE INVENTION

A wide variety of retroviruses are known. Also, it has been known that the transcriptional control elements of retroviruses lie within the part of the viral genome known as the long terminal repeat (LTR), more particularly within the U3 region of the LTR. See H. M. Temin, Cell, 27: 1, (1981); ibid, 28: 3 (1982).

In accordance with the present invention, it has been discovered that certain viruses, and certain factors coded for by segments of the genomes of those viruses, have the ability to multiply by many times the amounts of products produced by genes which operate under the control of the LTR segments of those viruses. This phenomenon, called trans-acting transcriptional regulation, is shared by a number of viruses, including the various types of human T lymphotropic viruses (HTLVs), and bovine leukemia virus (BLV). As used herein, the term "trans-activating retrovirus" is intended to mean those viruses which have trans-acting transcriptional regulation activity.

The human T lymphotropic viruses (HTLVs) are retroviruses associated with disorders of the OKT4+ (helper) subset of T lymphocytes.

HTLV Type I (HTLV-I) is the probable etiological agent of adult T cell leukemia (see, B. J. Poiesz et al., Proc. Natl. Acad. Sci. USA, 77: 7415 (1980); V. S. Kalyanaraman, et al., Nature (London), 294: 271 (1981); M. Robert-Guroff, et al., J. Exp. Med., 154: 1957 (1981); M. Yoshida, et al., Proc. Natl. Acad. Sci. USA, 79: 2031 (1982); and M. Popovic et al., Nature (London), 300: 63 (1982)).

HTLV Type II (HTLV-II) is an isolate originally derived from a patient with a T cell variant of hairy cell leukemia (see, V. S. Kalyanaraman et al., Science, 218: 571 (1982); I.S.Y. Chen, et al., Nature (London), 305: 502 (1983); E. P. Gelmann, et al., Proc. Natl. Acad. Sci. USA, 81: 993 (1984)).

Human T lymphotropic virus Type III (HTLV-III) has recently been identified as the probable etiologic agent of acquired immune deficiency syndrome (AIDS), a transmissible immunosuppressive disorder characterized by depletion of OKT4+ (helper) T cells (see, M. Popovic, et al., Science, 224: 497 (1984); R. C. Gallo et al., ibid. p. 500; J. Schupbach et al., ibid, p. 503; M. Sarngadharan, et al., ibid, p. 506). This agent has been assigned to the HTLV family based on its retroviral characteristics (ibid), the preference for magnesium of the reverse transcriptase (ibid), the specificity for OKT4+ T lymphocytes (ibid), the cross-reactivity of the p24 and envelope proteins with those of HTLV-I and HTLV-II (see, S. K. Arya et al., Science, 225: 927-930 (1984); and M. Essex et al., Science, 221: 1061 (1983)), and a distant relatedness at the nucleic acid level to HTLV-I and II (ibid).

In many respects, the diseases induced by bovine leukemia virus (BLV) resemble those induced by HTLV-I. The diseases have a long latent period that is sometimes preceded by lymphocytosis (Ferrer et al., Cancer Res., 34: 893 (1974); and Gallo et al., Blood, 60: 545 (1982)). In both diseases there is no chronic viremia in the target organs preceeding the disease and there is no preferred site of DNA integration of the provirus in the tumor cells (see, Paul et al., supra; Kettman et al., supra; Gregoire et al., supra; Gallo et al., supra; and B. Hahn et al., Nature, 305: 340 (1983)).

It has now been discovered that in cells which have been infected with trans-activating retrovirus, the rate of transcription of heterologous genes in vectors directed by the viral long terminal repeat sequences (LTR) is greatly augmented. It has further been discovered that transformation of a host cell with an appropriate vector which includes the portion of the viral genome of such viruses which provides the trans-acting transcriptional regulation, permits the trans-activation of heterologous genes, thus providing production of dramatically increased amounts of the desired gene product, without the necessity of using virus infected host cells.

### SUMMARY OF THE INVENTION

This invention results from the discovery of a novel phenomena in retrovirus transcription, namely transcriptional trans-activation. Described herein are novel genes, designated herein as the "tat" genes, which code for novel trans-activating transcription (tat) factors. Also described herein are certain cis-activated regions in the LTR section of the viral genome, referred to herein as tar regulatory elements, which are acted on by the trans-activating factors to produce the trans-activation effect.

One aspect of the present invention involves a method for stimulating the production of a gene product, which comprises: (a) inserting a trans-acting DNA segment into a host cell, the trans-acting DNA segment comprising a segment of the genome of a trans-activating retrovirus, which segment codes for a trans-acting factor; (b) inserting an expression vector into the host cell, the expression vector comprising the gene which codes for the gene product to be expressed and a cis-acting regulatory element which is responsive to the trans-acting factor produced by the trans-acting DNA segment; and (c) cultivating the host cell.

Further described herein are examples of methods and products utilizing the trans-acting transcriptional regulation phenomenon to augment the transcription and expression of homologous genes, such as HTLV envelope glycoprotein, and heterologous genes, such as a prokaryotic chloramphenicol acetyl transferase (CAT) gene, and the use of such genes to identify trans-activating viruses and factors, and to measure their effectiveness.

Use of the products and methods of the present invention substantially increases the rates and amounts of expression of gene products by recombinant DNA techniques. Gene product expression is typically at least about five time greater using the paired expression systems of the present than gene expression in the absence of such systems. Preferably the paired gene expression system of the present invention increases the amount of gene product expressed by a factor of at least about 30 times, more preferably at least about 500 times the amounts produced in the absence of the factors described herein. As disclosed further below, the present methods and products in particular instances have increased gene product production by a factor of greater than 1100.

### BRIEF DESCRIPTION OF THE FIGURES

Figures 1(a), (b) and (c) depict schematic representations and constructions of recombinant plasmids utilized in the present invention. The diagrams depict the region of the LTR's placed 5' to the CAT gene;
Figure 2(a), (b) and (c) illustrate the transient expression of the CAT gene directed by the LTR transcriptional elements. The graphs depict typical CAT assays over the time course indicated. All experiments were performed a minimum of 3 times with results differing by no more than 30 percent. Symbols represent CAT activity directed by the plasmids, pU3R-l (○), pSV2CAT (●), pV3-II (▭), pV3R-II ( 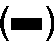 ), and pSVXCAT (△). Insets show actual autoradiograms of a CAT Assay and represent conversions obtained from one time period within the linear range of the assay;
Figure 2(d) illustrates the transient expression of the CAT gene in HTLV-III infected cells and uninfected cells; and
Figure 3 is a schematic representation of the HTLV-I viral DNA showing the relative location of the gag, pol and env genes, as well as the pX or tat gene. The tat gene causes trans-acting transcription when used in conjunction with HTLV LTR directed synthesis.
Figure 3(A) is a similar schematic representation of the HTLV-I viral DNA, showing the location of the segments of the tat gene, and of the Bam HI and Cla I restriction endonuclease sites used in analyzing that gene.
Figure 4 is a depiction of the deletion plasmids and expression activity thereof, used in deterimining the location of the trans-acting transcription factor on the HTLV-III genome.
Figure 5 is a depiction of the 3' region of the HTLV-III genome, together with the nucleotide and amino acid sequence of the trans-acting transcription factor.
Figure 6 is a depiction of the portion of the HTLV-III LTR which contains the cis-acting regulatory regions.

### DETAILED DESCRIPTION OF THE PREFERRED EMBODIMENTS

The LTR regions of the various HTLV's diverge markedly in sequence (see Shimotohno et al., supra, regarding the differences in LTR between HTLV-I and II). It is herein reported that the long terminal repeat elements of trans-activating retroviruses can, in the appropriate environment, promote the expression of heterologous genes. It has been discovered that species specific and tissue specific differences occur in the rate of heterologous gene expression under control of the HTLV LTRs. It has also been discovered that there are major differences in the activity of the HTLV-I, II and III and BLV transcriptional elements.

A most striking set of observations indicate that in cells infected by HTLV, trans-activating factors markedly stimulate transcription promoted by the HTLV LTR. This is true whether the gene which is associated with the LTR is homologous or heterologous, and is also true where less than all of the LTR is associated with the gene to be expressed. It turns out that there are minimal genetic segments of the LTR region which can operate in conjunction with the TAT factors to provide radically greater transcription and hence expression of the gene of interest. These segments are called TAR elements, and can be utilized to provide trans-activated response with or without homologous enhancers or heterologous enhancers. Preferably at least one enhancer is utilized in connection with the TAR element. The trans-acting transcriptional factors display some type specificity for activation of HTLV transcriptional elements.

The phenomenon of transcriptional trans-activation, and the ability of the LTR region of the HTLV and BLV viral genome are illustrated in the experimentation resulting in the results reported in Table 1. In those experiments, plasmids containing a prokaryotic gene, namely chloramphenicol acetyl transferase (CAT), included various trans-activating retrovirus LTR's as promoters. Similar experiments have been done with the rabitt B-globin gene. Other suitable genes for expression in accordance with the present invention include any products which are suitable for production by genetic manipulation techniques in a suitable host, e.g. heterologous genes coding for the interferons, insulin, human and other growth factors, or other expressible gene products, or homologous genes which code for useful viral products, such as the HTLV-I surface glycoprotein, useful as an antigen or vaccine.

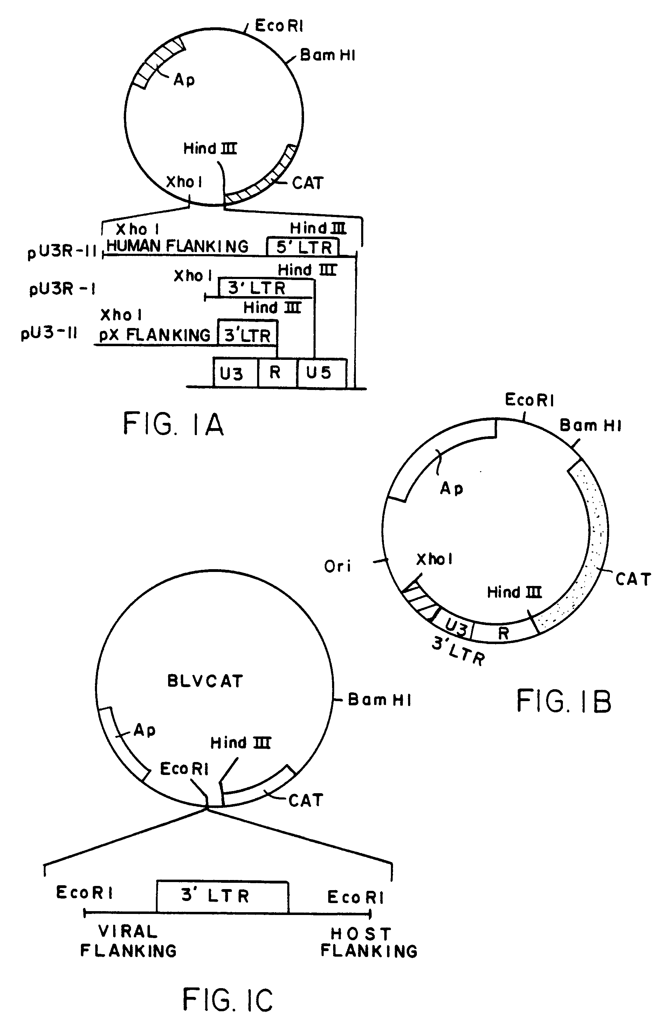

For example, the plasmid pU3R-III (construction shown in Figure 1(B), included the entire U3 and approximately 80 nucleotides of the R region from an HTLV-III cDNA clone was inserted 5' to the chloramphenicol acetyl transferase (CAT) gene (see, C. M. Gorman, et al., Mol. Cell. Biol., 2: 1044 (1982); C. M. Gorman, et al., Proc. Natl. Acad. Sci. USA, 79: 6777 (1982)). This plasmid (pU3R-III) was introduced into eukaryotic cells via transfection and the level of CAT enzymatic activity, which correlates with steady-state CAT messenger RNA levels (see, ibid; and M. D. Walker, et al., Nature (London), 306: 557 (1983)), was measured 48 hours post-transfection. Similar transfections were made with plasmids similarly constructed of using the CAT gene under the control of the LTR promoter region of HTLV-I (pU3R-I), HTLV-II (pU3R-II) and BLV (pBLV-CAT). Thus plasmids pU3R-I, pU3R-II and pBLV-CAT contain the HTLV-I, HTLV-II and BLV LTR's, respectively, located 5' to the bacterial chloramphenical acetyltransferase (CAT) gene (see Gorman, C. M. et al., Molec. Cell. Biol., 2: 1044-1051 (1982) ). See Figures 1(A), 1(B) and 1(C) for the constructions of these plasmids.

The CAT activity was normalized to that observed following transfection of pSV2CAT, a plasmid that contains the SV40 early region promoter 5' to the CAT gene (Gorman et al., supra), to control for differences in the ability of different cell types to take up and express foreign DNA.

The sequence for the LTR of the HTLV-I strain used for these studies is presented in Figure 1A (see, Seiki, M. et al., Proc. Natl. Acad. Sci. USA, 80: 3618-3622 (1983)). The sequence contains two 51 nucleotide long and three 21 nucleotide long imperfect repeated sequences. The results confirm that the U3 region of the HTLV LTR can serve as a promoter for heterologous gene transcription in non-lymphoid and lymphoid cells.

The results of the transfection experiments are shown in Table 1. The HTLV-III LTR functions as an efficient promoter in a variety of animal and human lymphoid and non-lymphoid cell lines. Apparently, the HTLV-III LTR, like the HTLV-I LTR, is not functionally restricted to lymphoid cells. There is no significant stimulation of CAT activity directed by pU3R-III in cells infected with HTLV-I, HTLV-II or BLV. The trans-acting transcriptional factors associated with HTLV-I, HTLV-II or BLV do not stimulate gene expression directed by the HTLV-III LTR.

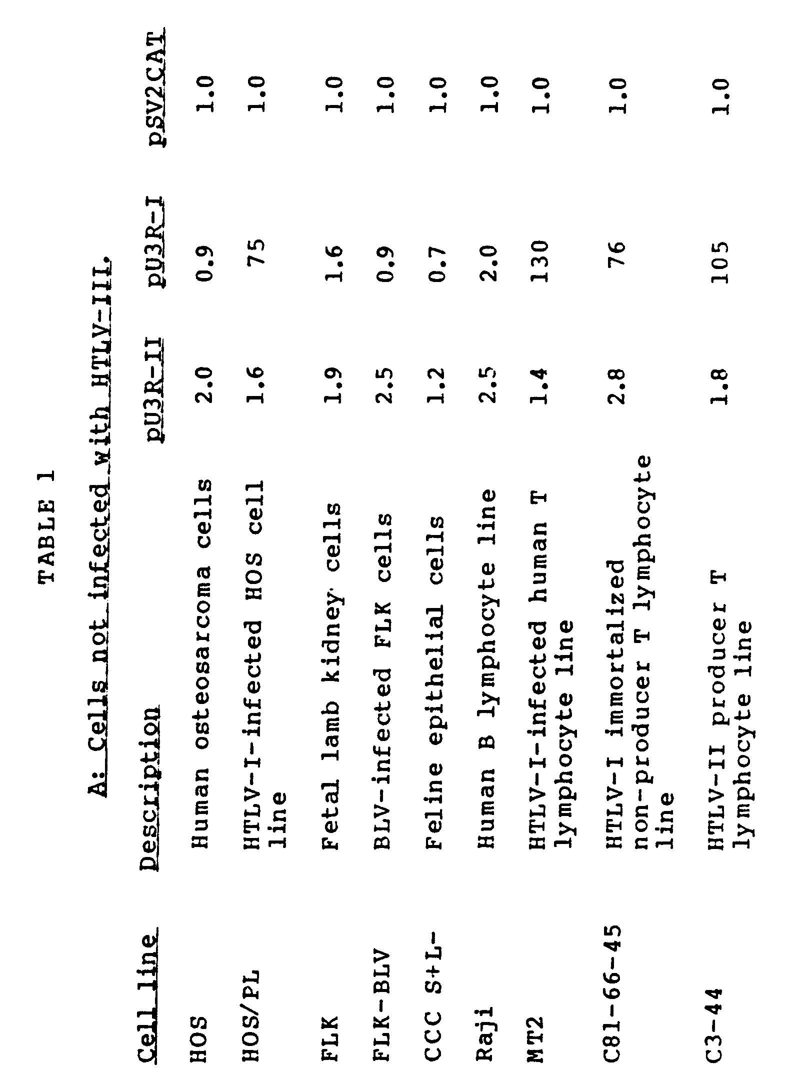

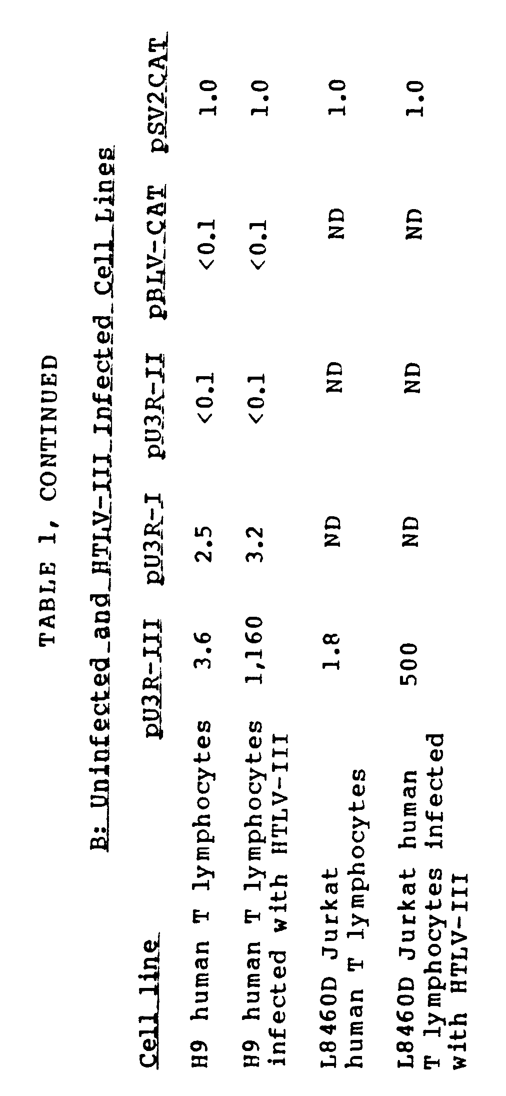

The above results indicate the dramatic increases which can be obtained in production of heterologous gene products in acordance with the present invention. For example, the use of HTLV-I LTR controlled expression vector pU3R-I in HTLV-I infected HOS and human T lymphocytes (MT2) cells resulted in approximately 75-fold and 130-fold increases in production of CAT enzymatic activity, respectively. Similarly, the use of HTLV-III LTR controlled expression vector pU3R-III in HTLV-III infected H9 and human T lymphocytes (L8460D) cells resulted in approximately 1,160-fold and 500-fold increases in production of CAT enzymatic activity, respectively. Similar results can be obtained for homologous gene products, e.g., HTLV env glycoprotein. Also, similar increases in gene production in non-infected cells by using HTLV LTR directed expression vectors in conjunction with vectors only expressing the trans-activating factors is discussed more fully below.

The genome of bovine leukemia virus (BLV) contains the LTR, gag, pol, and env sequences characteristic of all retroviruses (S. Oroszlan et al., supra). In addition, like the human T lymphotropic virus (HTLV), the BLV genome has a long open reading frame (LOR) region of approximately 1600 nucleotides 3' to the envelope gene (W.A. Haseltine et al., Science, 225: 419 (1984)).

To test the transcriptional capabilities of the BLV LTR sequences, plasmids were constructed in which the BLV LTR was placed 5' to the bacterial chloramphenicol acetyl transferase (CAT) gene (Figure 1(C)) (Gorman et al., supra). To assay for transcriptional activity the recombinant plasmids were introduced into eukaryotic cells via transfection (Graham et al., supra). Levels of CAT enzymatic activity correlate closely with levels of CAT messenger RNA, thereby providing a measure of the ability of the sequences 5' to the CAT gene to promote transcription (Gorman et al., supra; S.L. McKnight et al., Nature (London), 306: 557 (1983)).

The ability of the BLV LTR sequences to function as transcriptional elements in uninfected murine fibroblast and human epithelial lines was tested. These data are summarized in Table 2.

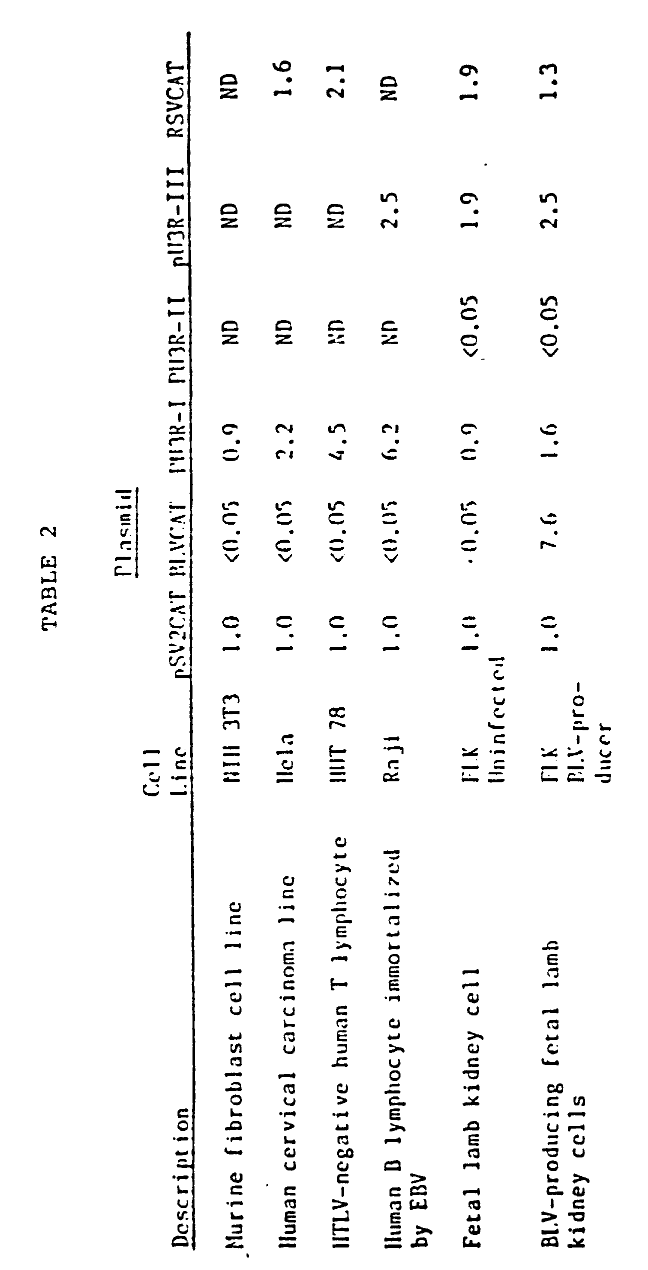

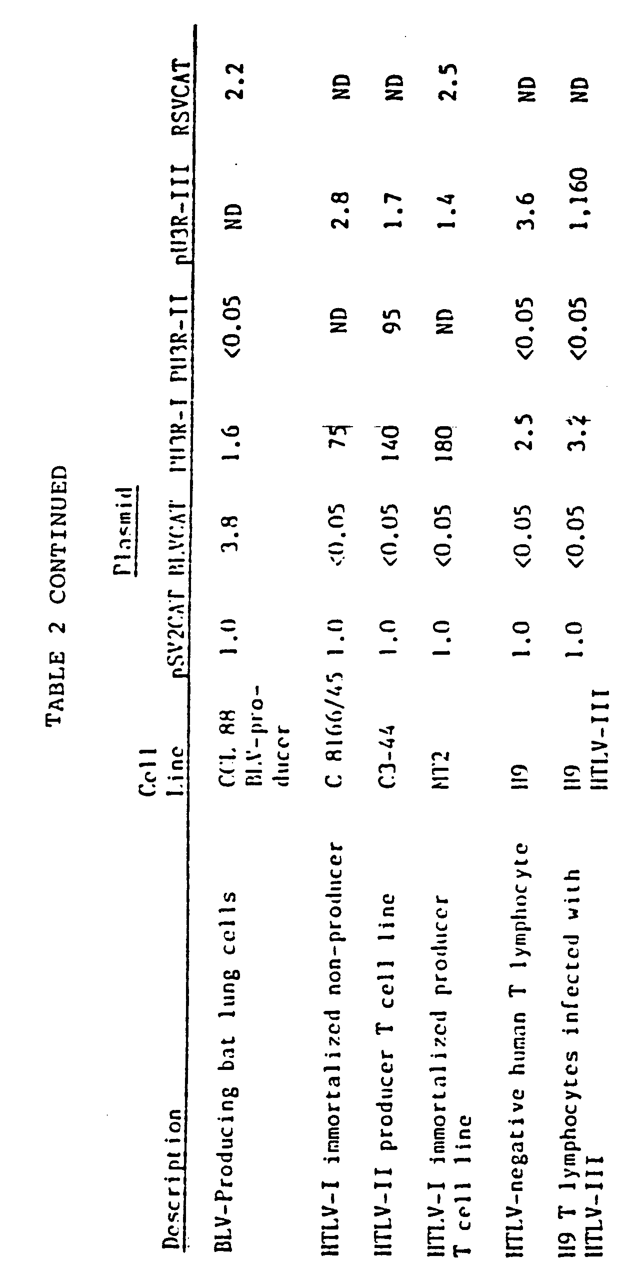

Parallel experiments were done using several other plasmids in which the CAT gene was under control of other promoter sequences. Upon transfection these additional plasmids all yielded appreciable levels of CAT activity (Table 2), indicating that the transfection procedures used resulted in efficient uptake and expression of DNA. No CAT activity was detected in extracts prepared from those cells that were transfected with the plasmid containing the BLV LTR sequences. Similar results were obtained following transfection of the human T and B lymphocytes (Table 2). These results show that the BLV transcriptional elements do not function as a transcriptional promoter in these cells.

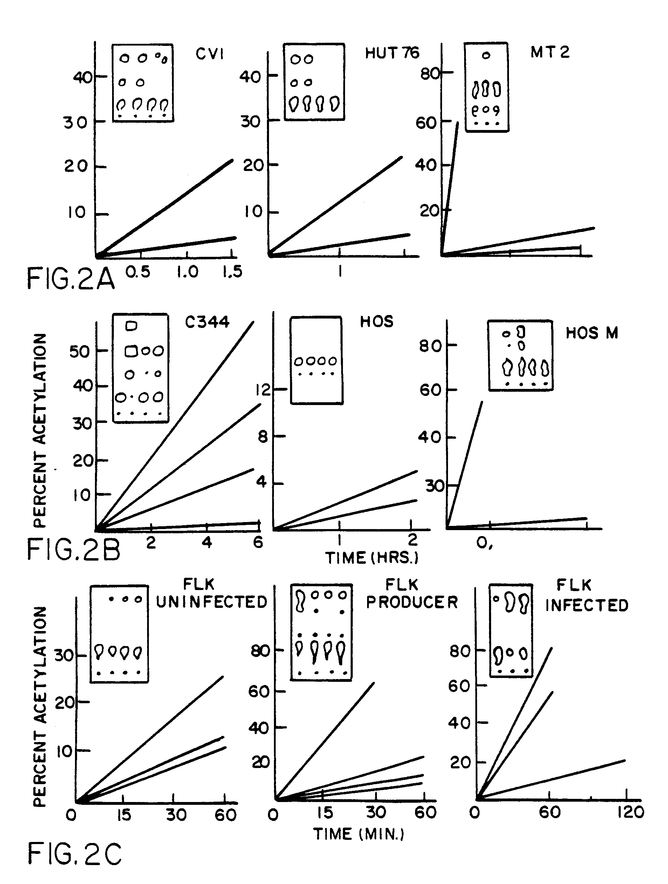

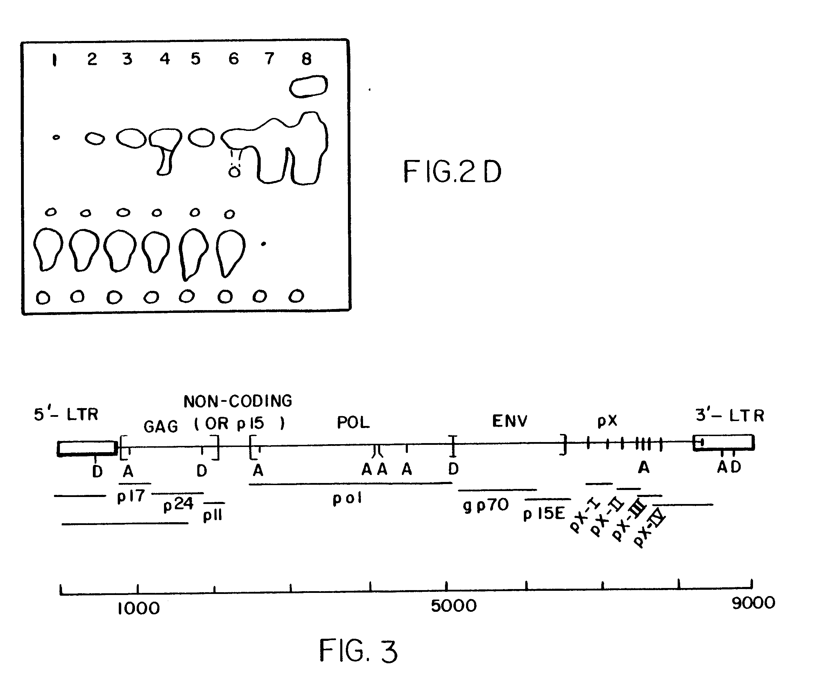

The ability of the BLV LTR sequence to function as a transcriptional element in BLV infected and uninfected fetal lamb kidney cells was also examined. The CAT activity directed by the BLV LTR sequences was not detected in the uninfected cells, suggesting that the BLV LTR was inactive in these cells. In contrast, a high level of CAT activity was detected upon transfection of the BLV infected producer cells (Table 2 and Fig. 2). The infected cell line used for this experiment produced BLV virons as demonstrated by high reverse transcriptase levels (data not shown). Similar results were obtained using additional uninfected and BLV infected matched cell lines. Cell lines used were uninfected bat lung cells (CCL88) and a clonal isolate of the CCL88 line that produced BLV (D.C. Graves et al., Cancer Res., 36: 4152 (1976)) and FLK cells that were infected with BLV six to eight days prior to transfection (Table 2 and Fig. 2).

From these experiments we conclude that trans-acting factors, either encoded by the virus of virally induced, activate BLV LTR-controlled gene expression. This effect most likely occurs at the level of transcription (McKnight et al., supra).

Gene expression directed by the HTLV-I, HTLV-II and HTLV-III LTR sequences is augmented by trans-acting factors present in HTLV infected cells. Also tested was whether or not these factors could activate the BLV transcriptional control sequences.

As indicated in Table 2, the trans-acting factors present in the HTLV-I, HTLV-II, and HTLV-III infected cell lines were unable to activate BLV LTR-directed CAT gene expression. Similarly, factors present in the BLV infected cells were unable to increase CAT gene expression directed by the different HTLV-LTR sequences. This suggests that the trans-acting factors for HTLV-I, HTLV-II and HTLV-III are not functionally interchangeable with those factors which activate the BLV LTR sequences; this is not surprising given the lack of primary sequence homology between the HTLV and BLV LTR sequence (D. Couez et al., J. Virol., 49: 615 (1984); A. Tsimanis et al., Nuc. Acid. Res., 17: 6079 (1983); and J. Sodroski et al., Proc. Natl. Acad. Sci. U.S.A., 81: 4167 (1984)).

The major determinant for efficient function of the trans-activating retrovirus transcriptional elements appears to be the presence of the induced trans-activating factors, which are mostly specific to the LTR promoters of their own type. Thus trans-activated CAT gene expression directed by the plasmid that contains the BLV LTR was only observed in BLV-infected cells. In this respect, BLV resembles HTLV-II, since the efficient activity of the HTLV-II LTR appears to be restricted, for the most part, to HTLV-II infected cells. The HTLV-I LTR directed transcription, on the other hand, appeared to be efficiently conducted in at least some cells which were only infected with HTLV-II.

The phenomenon of trans-activation and the presence of a LOR region distinguish both BLV and HTLV from other non acute retroviruses. It has been found that trans-activation of the HTLV LTR is mediated by the protein product of the HTLV tat gene. As the genome of BLV contains a LOR region 3' to its envelope gene (W.A. Haseltine et al., Science, 225: 419 (1984)) it is therefore proposed that the BLV LOR protein product mediates transcriptional -activation of the BLV LTR. A two kilobase message capable of encoding such a protein has been detected in BLV infected cells (Ghysdael et al., J. Virol., 29: 1087 (1979)).

### IDENTIFICATION OF TRANS-ACTIVATING VIRAL GENES

### A. Location of the tat Gene in HTLV-I and HTLV-II

The sequence of the 3' terminus of the human T lymphotropic virus Type II (HTLV-II) was determined and compared to the 3' termini of the genomes of HTLV-I, bovine leukemia virus (BLV), and mouse mammary tumor viruses (MMTV). All of these viruses contain long open reading frames sufficient to encode proteins in the molecular weight region of 38-40,000.

The nucleotide sequence of 1,557 bases of the 3' terminal region of the HTLV-II can be divided into two domains. One domain, 547 nucleotides long, is located at the 5' end of the sequence and has either no or very little sequence similarity to the corresponding regions in HTLV-I. This domain is herein called the non-conserved region (NCR). A second region, 1,011 nucleotides long, comprises the 3' portion of this region. This sequence is very similar to that of HTLV-I. The sequence can be aligned with that of HTLV-I and is identical at 716 of 1,011 nucleotides (70%) homology.

The perimeters of the 1,011 nucleotide long sequence of the HTLV-II genome correspond precisely with a single long open reading frame capable of encoding a polypeptide 337 amino acids long. A corresponding sequence of HTLV-I also encompasses a single long open reading frame capable of encoding a polypeptide 359 amino acids long.

Proteins encoded by HTLV-I and II are of approximately the same length and are identical in 278 of 337 of the amino acids (82% identity). The degree of similarity of these two proteins is even more striking if conservative amino acid substitutions are considered (95% similar). The distribution of hydrophylic and hydrophobic regions of these proteins is remarkably similar.

Several other long open reading frames exist in the 3' regions of both Type I and Type II HTLV. None of these are found to be common to both HTLV-I and HTLV-II. No regions of predicted protein sequence similarity could be found upon comparison of the coding capability of the HTLV-I and II viruses in the 3' region in any reading frame with the notable exception of the 3' terminal sequence discussed above. In particular the pX1, X2 and X3 long open reading frames noted by Seiki, (Seiki et al., infra) have no counterpart within the 3' sequence of HTLV-II, whereas the pX4 corresponds to the carboxyl terminus of the long open reading frame noted above. An 11 base pair deletion in the NCR region of HTLV-C relative to that of HTLV-I, apparently has no effect on the biological activity of the virus.

The 3' terminal region of HTLV-I and II contain a new gene that encodes a protein that is of a molecular weight of about 42Kd in the case of HTLV-I, and about 38 Kd in HTLV-II. These proteins are greater than ninety percent homologous at the amino acid level, and are located predominantly in the nuclei of infected cells. A protein of molecular weight 40-42,000 in HTLV-I-infected cell lines has been noted that is recognized by sera of persons infected with HTLV-I, but not by control human sera.

It has now been discovered that these long open reading frames in HTLV-I and II code for the trans-activating transcriptional factors for HTLV-I and II, respectively. These genes (sometimes previously referred to as PX or x-lor genes) are therefore referred to herein as the tat genes for HTLV-I and HTLV-II.

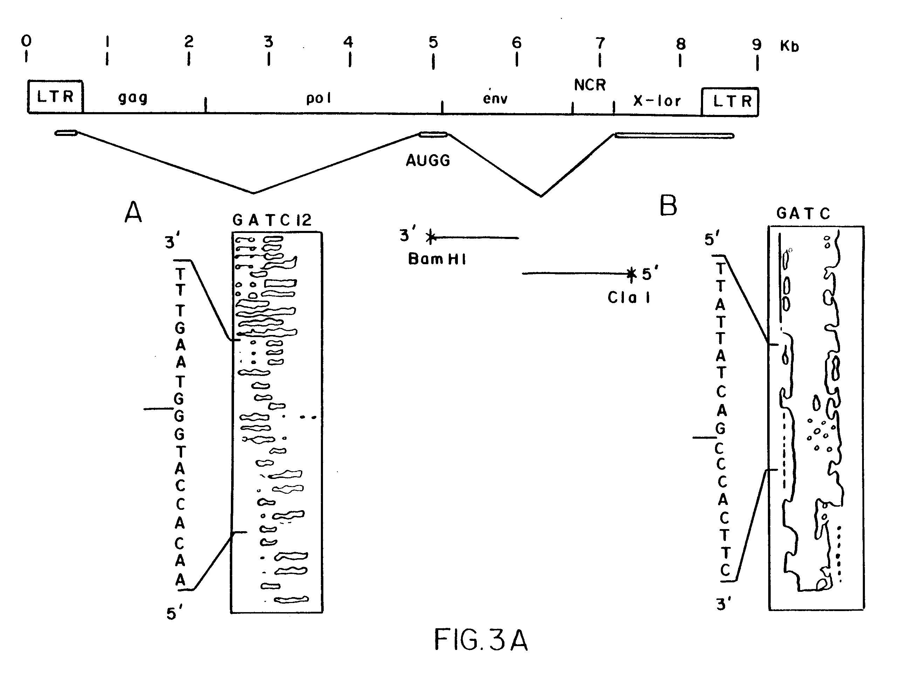

The HTLV-I and II tat genes lack initiator methionine codons, but exhibit splice acceptor consensus sequences at their 5' ends. The tat gene product proteins are made from spliced 2 kilobase messages that often contain, in addition to LTR- and tat -homologous regions, sequences surrounding the pol-env junction. To gain insight into the composition of the natural tat gene product protein, we mapped selected splice donors and acceptors in the HTLV-I-immortalized C81-66-45 cell line. This cell line exhibits only 2 kilobase HTLV-related messenger RNA and expresses only the HTLV-I 42 tat gene product protein, which is detectable by antisera from patients having adult T cell leukemia/lymphoma (ATLL). The C81-66-45 mapping identified a splice donor consensus sequence located immediately 3' of the HTLV-I env gene start codon, and a splice acceptor consensus sequence located 172 bp 5' to the Cla I site in the tat gene, in a position identical to that previously determined for the codable message in the HTLV-I long open reading frame. A probable scheme for production of the natural tat message and protein is presented in Figure 3. Via a dual splicing event, the HTLV env gene contributes its initiation codon plus a single guanosine residue to the tat gene, resulting in an in-frame coding sequence.

Knowledge of the splicing pattern for the tat message suggested that the natural tat products could be expressed in eukaryotic cells by placing the HTLV-I LTR 5' to the env-X regions of the HTLV-I and HTLV-II genomes. Such plasmids can then express envelope proteins, tat proteins, and other products from the 3' half of the HTLV genome. To establish that the trans-activating tat factor could be expressed alone and stimulate HTLV LTR-promoted expression vectors, a plasmid (pCATLOR_{II}) was designed to express only a fusion protein consisting of nine amino terminal residues encoded by the bacterial chloramphenicol acetyltransferase (CAT) gene, and the product of the entire HTLV-II tat gene extending from the splice acceptor to the termination codon. This hybrid gene was cotransfected into various cell lines with the plasmids pU3R-I and pU3-II. Cotransformants of CATLOR_{II} with pU3R-I evidenced efficient transcription in human T lymphocytes (HUT78), simian kidney cells (COS-l) and murine fibroblasts (NIH3T3). The ability of such plasmids to affect the rate of gene expression directed by the HTLV-I and II LTR sequences was examined.

The fusion product or the plasmid CATLOR_{II} produced evidence of trans-activation in the form of overproduced chloramphenicol acetyltransferase when cotransfected with plasmids in which the CAT gene was placed under the control of HTLV LTR's. This, plus the results obtained with other indicator plasmids, indicates that the gene product of the HTLV-II long open reading frame (tat gene), which would be part of the fusion product of the CATLOR_{II} plasmid, is the trans-activating factor. It also establishes that the HTLV trans-activating factors can be utilized to produce desired gene products. Such trans-activating factors can be included in the desired host cell by infection, by stable incorporation into the host genome following transfection, or transiently, by cotransfection with the LTR-controlled expression or other vector bearing the gene of interest. The tat gene can also be included on the expression vector itself, provided that the size of the vector does not exceed the capacity of the host cell for uptake and expression.

Another use of the trans-activating factors disclosed herein is in the immortalization of cell lines. Cellular immortalization by HTLV-I is mediated either directly or indirectly by the HTLV tat product, possibly via trans-activation of cellular genes involved in lymphocyte growth control. As the disease characteristics together with the phenomenon of trans-activation distinguish BLV and HTLV from other non acute retroviruses, it appears that the product of the HTLV tat gene plays an important role in the transformation process.

### B. Location of tat gene for HTLV-III

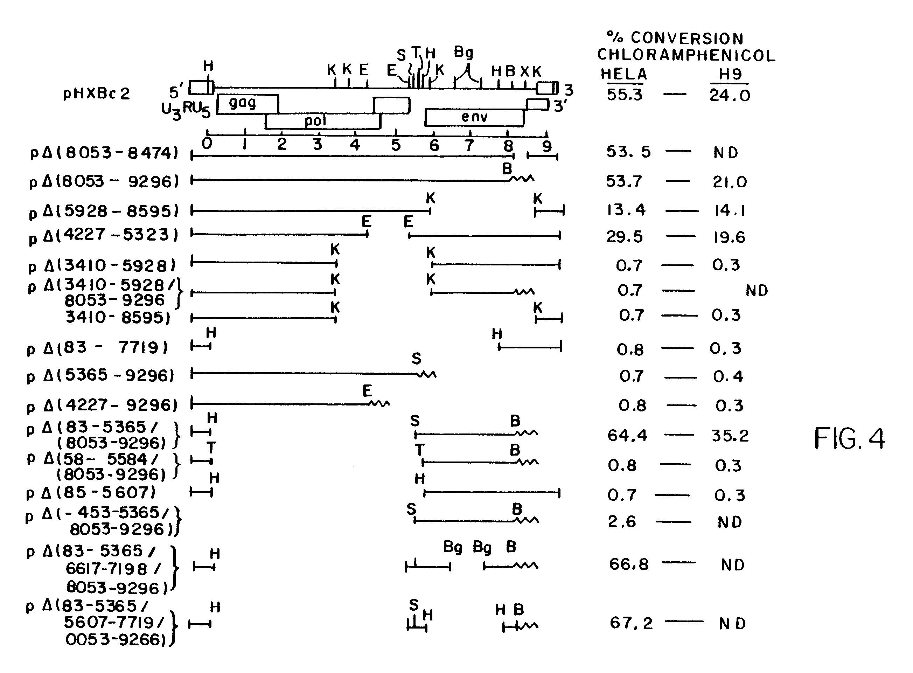

In order to determine what regions ofthe HTLV -III are necessary for determining trans activation, deletions were introduced in the HTLV-III proviral genome. The deleted plasmids are named using the nucleotide numbers of the deletion endpoints based on the sequence of the HTLV-III genome by Ratner et al., Nature, 313:277 (1985). Deletion mutants carried on a plasmid (pHXBc2) were tested for trans-activating ability in cotransfection experiments similar to those described above. Hela cells were used as recipients for testing the deletions, since the low intrinsic activity of the HTLV-III LTR in this cell line allows a sensitive assessment of the presence or absence of trans-activation. The deletion plasmids are shown in Figure 4, along with the relative amounts of CAT expression obtained using them. H9 human T lymphocytes were also used for some of the experiments.

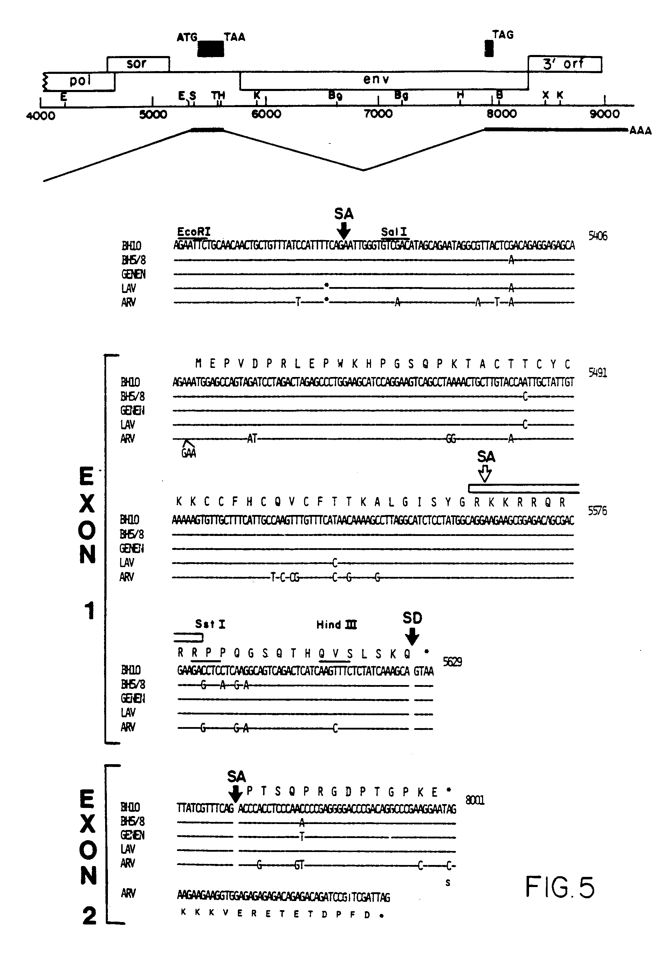

The result of this study is the identification of the portion of the HTLV-III genome which encodes the trans-activation factor for HTLV-III. This portion of the genome corresponds to that defined by the deletion plasmid pD(83-5365/5607-7719/8033-9266). The predicted amino acid sequence ofthe potential product of the tat region of the HTLV-III genome is shown in Figure 5. amino acid residues 49 through 57 are noteworthy in that they are extremely basic in nature. Such arginine/lysine-rich regions have been found in several nuclear proteins exhibiting the ability to bind to nucleic acid. This basic stretch is invariant amont the various HTLV-III isolates sequenced, suggesting that it may be conserved in order to preserve function.

The tat gene and its product in the case of HTLV-III can be used in the same manner as that of HTLV-I and HTLV-II, in order to obtain large amounts of production of gene products, e.g. by infection of the host cell, by permanent addition of the tat gene to the host cell genome, by cotransfection with HTLV-III LTR promoted expression vectors, or by incorporation into such vectors.

### LOCATION OF CIS-ACTING REGULATORY ELEMENTS THAT RESPOND TO TRANS-ACTING FACTORS

### A. Location of Cis-Acting Regulatory Sequences In HTLV-I Long Terminal Repeat.

The long terminal repeats that flank the integrated provirus in HTLV viruses contain recognition signals that interact with the host RNA polymerase. The signals include initiation sites for the polymerase, promoters and sequences called enhancers that govern the rate of transcription at a nearby promoter. In addition to enhancer and promoter elements, we expected to find that the HTLV-I LTR contained cis-acting regulatory elements responsive to the trans-acting regulatory factors present in HTLV-I infected cells.

To identify cis-acting regulatory elements 5' to the "TATA" sequence in the LTR, a series of 5' deletion mutants were constructed. Deletions were made in plasmid pU3R-I, described above and in Figure 1, which contains HTLV-I LTR sequences 5' to the structural coding sequences for the bacterial CAT gene. Deletion plasmids were transfected into eukaryotic cells and CAT enzyme activity was measured from cell extracts prepared 48 hours post transfection.

The effect of deletions upon the rate of transcription in non-lymphoid and lymphoid cells is shown in Figure 2c. The first deletion of 70 nucleotides resulted in a substantial loss of transcriptional activity. The activity was partially restored by deletion of an additional 10 nucleotides (Figure 2c). A negative regulatory element thus may be located between positions -294 and -286 of the U3 sequence.

A surprising discordance in the effect of deletions on transcription in Hela cells as compared to transcription in lymphoid cells was observed for the deletions -262 and -240. These deletions reduce levels of CAT activity in Hela cells by about ten fold, but reduce the level of transcription by only two-fold in lymphoid cells. This observation suggests that a sequence required for efficient transcription in non-lymphoid cells is located between nucleotides -286 and -262 and that such a sequence is not required for efficient transcription in some lymphoid cells. Deletion of an additional 10 nucleotides to position -230 substantially decrease the level of transcriptional activity in all cells examined. The latter deletion results in removal of almost two thirds of the upstream 51 bp repeat and an entire 21 bp repeat. Thus, although the 51 bp and 21 bp imperfect repeats are duplicated and triplicated respectively within the LTR, the data imply that the 5' most repeat sequence is required for complete transcriptional activity.

Analysis of the deletion study also provides information as to the 5' boundary of the trans regulated region. The level of CAT gene expression is approximately 75-150 fold higher (when normalized against the SV40 early region promoter element) in HTLV infected as compared to uninfected cells. This ratio remains constant for the deletions extending through position -230 (Figure 2c). Thus, viral-associated trans regulation is not dependent upon sequences between the 5' end of the LTR and position -230. Furthermore, as deletion pC55 which retains the 55 nucleotides upstream of the transcription start site directs little CAT activity we surmise that some sequences required for trans-activation have been removed.

Since repetitive sequences 5' and distant to the TATA box clearly play a role in the transcriptional activity of the HTLV-I LTR, we sought to determine whether such sequences within the U3 region might serve as an enhances element, a sequence capable of increasing the rate of transcription on a heterologous promoter independent of orientation (see, Benoist, C. et al., Nature, 290: 304-310 (1981); Banerji, J., et al., Cell, 27: 299-308 (1981); and Gruss, P. et al., Proc. Natl. Acad. Sci. USA, 78: 943-947 (1981)).

The portion of the LTR extending from the 5' terminus to a position 35 nucleotides upstream to the TATA initiation sequence was inserted 5' to the SV40 early region promoter that lacked its own enhancer element in pSVIXCAT. Plasmids were constructed that included either one or two copies of the U3 sequence in the same transcriptional orientation or a single copy in the reverse orientation (Fig. 1c).

Levels of CAT activity upon transfection of Hela cells are shown in Figure 2b. Very little activity was detected in the absence of the HTLV U3 sequence. However, CAT gene expression was either 45 or 130 times higher for the plasmids that contained either one or two copies of the U3 sequence respectively. The level of CAT activity was 13 times that of pSV1CAT when the U3 sequence was positioned in the reverse orientation. Similar results were obtained following transfection of these DNAs into other non-lymphoid and lymphoid cells, some of which contain the HTLV associated trans-acting factor (Table 3). Sequences present within this portion of the LTR were thus concluded to must function as efficient transcriptional enhancers.

**TABLE 3**

| Relative CAT Activity % | | | |
|---|---|---|---|
| Cell Line | | | |
| Enhancer | Plasmid | Hela | C81-66-45 |
| HTLV | pHE3CAT | 1.0 | 0.15 |
| | pHXICAT | 31 | 3.20 |
| RSV | pRESl | 2 | ND* |
| | pREHl | 7 | 1.2 |

| | | | |
|---|---|---|---|
| *ND, not done. | | | |

Table 3 represents the comparative activity of HTLV and SV40 promoter sequences. The recombinant plasmids bearing the HTLV and RSV enhancer sequences 5' to either the HTLV or SV40 promoter sequences were transfected into the indicated cell types. CAT assays were performed after a 48 hour incubation. The values shown represent the percent of CAT activity as normalized to the activity present in similar cells transfected with the plasmid pU3R-I.

This enhancer effect is similar in all cells examined and is not restricted to cells of lymphoid origin. There does appear to be some preference for the transcriptional orientation as has been observed for other retroviral enhancer elements (see, Laimins, L. A. et al., J. Virol., 49: 183-189 (1984) and Fromm, M. et al., Mol. Cell. Biol., 3: 991-999 (1983)).

Additionally, although this region does contain repeat elements that might be formally analogous to the repeat sequences observed in other viral enhancers, this region does not contain sequences homologous to the consensus core sequences observed 5' to many eukaryotic and viral promoter elements (see Weiher, H. et al., Science, 219: 626 (1983) and Laimins, L. A. et al., Proc. Natl. Acad. Sci. USA, 79: 6453-6457 (1982)). Other sequences present within the viral LTR must therefore perform this function.

To determine if the region adjacent to the TATA initiation sequence can serve as a promoter for the heterologous enhancer elements plasmids were constructed that contained that portion of the U3 region extending 35 nucleotides 5' to the TATA sequence and through the RNA initiation sequence (Figure 2). Plasmids were also constructed that contained 5' to this sequence the U3 region of HTLV (in the reverse orientation) or the U3 region of Rous sarcoma virus (RSV) lacking the TATA initiation sequence. Hybrid plasmids that contained the SV40 early promoter region and U3 enhancer regions of HTLV and RSV were also constructed to permit comparison of the promoter functions of HTLV and SV40.

Relative to the plasmid containing the HTLV enhancer and SV40 promoter region CAT gene expression was 20-30 fold higher in cells transfected with the plasmid bearing the HTLV promoter and enhancer sequences (Table 3). The increased activity is not a function of the reversed HTLV enhancer juxtaposed to its own promoter. When the RSV enhancer sequences were positioned 5' to both the HTLV and SV40 promoter sequences, the construct that contained the HTLV promoter was at least three fold stronger than the analogous RSV enhancer construct (Table 3). This observation indicates that the region of the HTLV sequence around the TATA box can serve as a promoter for a heterologous enhancer element.

Transfection of C81-66/45 lymphoid cells with these plasmids yields levels of CAT activity similar to that obtained in Hela cells (Table 3). Thus, although the hybrid transcription regions as efficient promoters, they are not responsive to the trans-acting factors present in these cells. This complete functional region regulated in trans is therefore not present between -35 and + 315 of the HTLV LTR.

The data presented here also provide definition of some of the functional domains of the U3 portion of the HTLV-I LTR. Although this region lacks those sequences that resemble the consensus sequence found in other enhancer elements, the region of the LTR located between -335 and -35 does function as a transcriptional enhancer element. Furthermore, the HTLV enhancer demonstrates no detectable tissue preference as does the enhancer of the mouse immunoglobulin heavy chain gene (see, Gillies, S. D. et al., Cell, 33: 717-728 (1983) and Banerji, J. et al., Cell, 33: 729-740 (1983)) and some other retroviruses (see Laimins et al., al., supra and Linney, E. et al., Nature, 308: 470-472 (1984)).

These data suggest that the sequence present between positions -286 and -262 imparts a regulatory function in non-lymphoid cells. This possibility is intriguing as the LTR of HTLV-II, which lacks this sequence (see, Sodroski, J. et al., Proc. Natl. Acad. Sci. USA, 81: 4617-4621 (1984) and Shimotohno, K. et al., Proc. Natl. Acad. Sci. USA, 81: 1079-1083 (1984)), is not an efficient promoter in non-lymphoid cells (see Chen, I.S.Y. et al., Nature, 309: 276-279 (1984)). These data also demonstrate that the region activated in trans is not present in its entirety between positions -35 and -385 nor between positions -35 and +315. This suggests that the trans regulated region extends through these two regions.

### B. Location of Cis-Acting Regulatory Sequences In HTLV-III Long Terminal Repeat.

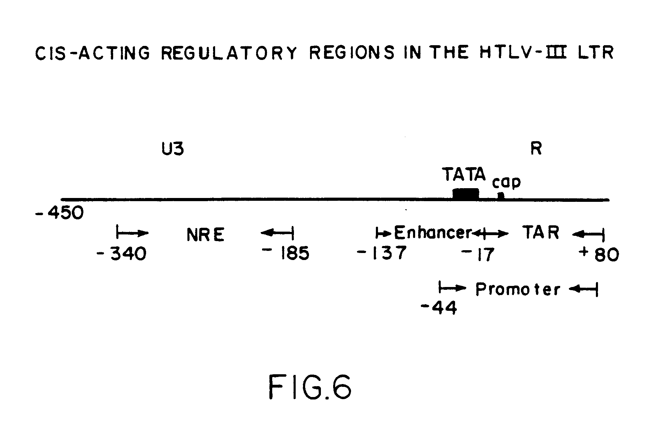

Using similar techniques, we determined the location of the cis-acting regulatory elements within the HTLV-III LTR. The various elements found are shown in Figure 6. This LTR contains an enhancer element, a promoter, a region necessary for the greatly increased level of HTLV-III LTR directed gene expression in virus infected cells, and a region that negatively regulates gene expression directed by the HTLV-III LTR and heterologous promoters.

The HTLV-III enhancer, located between nucleotides -137 and -17 (cap=+1), lacks both tandem repeat elements and core consensus sequences typical of many viral and cellular enhancer sequences. It resembles the HTLV-I enhancer that also lacks core consensus signals. The ability of the enhancer to functionally complement the activity of heterologous promoter sequences in lymphoid as well as non-lymphoid cell lines is noteworthy. The ability of the HTLV-III enhancer to function in many cell types leads us to conclude that it is unlikely that the tropism of HTLV-III for OKT4+ cells can be attributed to function of the LTR alone.

The enhancer elements do not themselves respond to virus associated trans-acting regulatory factors. In this respect they resemble the enhancer of HTLV-I, but differ from those of HTLV-II and BLV, that do respond, albeit weakly to those factors associated with infection by the hhomologous virus.
The sequences surrounding the site of genomic RNA initiation can serve as promoter sequences that respond to both homologous and heterologous enhancer sequences. These sequences cannot, however, in the absence of functional enhancers, respond to viral associated trans-acting regulatory factors. The sequences located between -17 and +80 are apparently sufficient for the responsive phenotype. These sequences do not overlap with those of the HTLV-III enhancer and are not co-terminal with the promoter sequences. For this reason we believe that this region contains a novel type of regulatory element, the TAR element, for trans-acting responsive or target element.

Identification of a sequence located at the 5' end of the HTLV-III LTR that negatively affects the level of gene expression directed from homologous as well heterologous enhancer promoters was unexpected. This sequence exerts a negative efect on the level of CAT gene expression from a distance and irrespective of orientation. It is noteworthy that the NRE sequence down regulates the HTLV-III LTR directed gene expression in both infected and in uninfected cells. The effect of the NRE is opposite that of an enhancer element.

Although emphasis has been placed on the tat and tar elements and their use in production of gene products, other elements may be included in the expression systems described herein to change or improve their functions, as will be appreciated by the skilled in this art. For example, the expression and/or activation vectors described herein may include other elements, such as polyadenylation signals, one or more selection markers, such as neomycin resistance, GPT resistance, or the like. Additional enhancer elements may be included, for example, to broaden the range of suitable hosts. Other modifications will be readily apparent to the skilled in the art.

The present invention will be further illustrated by the following examples. The examples are provided to aid in the understanding of the invention and are not to be construed as a limitation on the scope of the claimed invention. All recombinant DNA techniques, including use of XhoI and Hind III synthetic DNA linker are according to standard procedures.

The vectors of the present invention can be in the form of plasmids, or viral vectors, such as those produced in accordance with the procedure described by Mann et al., Cell, 33: 153 (1983).

Various cell lines differ in their ability to take up and express transfected DNA. In each experiment the CAT activity directed by the plasmids that contained HTLV sequences was normalized to that of plasmids that contained the SV40 enhancer promoter elements. The SV40 transcriptional elements have been shown to function in a wide variety of cell types and have been utilized as relatively neutral reference promoters in similar studies (Gorman, et al., supra; and M. D. Walker, et al., Nature, 306: 557 (1983)).

### EXAMPLE 1

### Construction of HTLV-CAT Recombinant Plasmids

The transcriptional control elements of animal retroviral LTRs are contained within the U3 region (see, H. M. Temin, Cell, 27: 1 (1981); and H. M. Temin, Cell, 28: 3 (1982)). Since the unusually long R region of the HTLV LTR might play a role in transcriptional regulation, the entire U3 and R regions of HTLV-I were inserted 5' to the chloramphenicol acetyltransferase (CAT) gene (pU3R-I and pU3R-II) (see Figure 1 and C. M. Gorman, et al., Mol. Cell. Biol., 2: 1044 (1982); C. M. Gorman, et al., Proc. Natl. Acad. Sci. U.S.A., 79: 6777 (1982)). A third plasmid that contained only the U3 and a portion of the R region of HTLV-II was also constructed, pU3-II (Fig. 1).

The activity of the CAT gene in transient transfection assays was compared to the activity of other plasmids that contained either the entire SV40 enhancer-promoter region (pSV2CAT), the promoter of SV40 without the 72 base repeat regions that comprise the SV40 enhancer (pSVIX CAT), or the entire LTR of Rous sarcoma virus (pRSVAT) located 5' to the CAT gene (see, C. M. Gorman, et al., Mol. Cell. Biol., 2: 1044 (1982); C. M. Gorman, et al., Proc. Natl. Acad. Sci. U.S.A., 79: 6777 (1982)).

To test the transcriptional activity of the inserted HTLV sequences, the plasmid DNA was introduced into cells via transfection using either the calcium phosphate or the DEAE-dextran methods (see, F. L. Graham et al., J. Virology, 52: 456 (1973); and C. Queen et al., Cell, 33: 729 (1983), respectively).

### EXAMPLE 2

### Expression of the CAT Gene in Fibroblast and Epithelial Cell Lines

The ability of the LTR sequences of HTLV-I and II to act as transcriptional elements in fibroblast and epithelioid cells of mouse, simian, and human origin was tested below.

The data of Table 4 and Figure 2 show that in (NIH 3T3) murine fibroblasts (see, G.J. Todaro et al., J. Cell Biol., 17: 299 (1963)), the activity of plasmids that contain the LTR sequences of HTLV-I was comparable to that of pSV2CAT and much higher than the level of the plasmid that contains the SV40 promoter sequence alone. The level of CAT activity in cells transfected with pRSV CAT was highest in this cell line. The HTLV-I sequences also yielded appreciable levels of CAT activity upon transfection into simian (CV-I) cells (see, F.C. Jansen et al., Proc. Natl. Acad. Sci. U.S.A., 53: 53 (1964)), although it was lower than that observed for the pSV2CAT plasmid. The LTR sequences of HTLV-II directed very low levels of CAT activity in both of these murine and simian cells lines. These experiments demonstrate that the LTR sequences of HTLV-I can function as efficient transcriptional elements in cells of different species, and that they exhibit markedly enhanced promotional strength in these cell types as compared to the HTLV-II LTR sequences.

**TABLE 4**

| Relative CAT Activity ^{a} | | | | | | |
|---|---|---|---|---|---|---|
| Cell line | Description | pSV2CAT | pU3R-I | pU3R-II | pU3R-II | RSVCAT |
| CV1 | Simian, fibroblast cell line | 1.0 | 0.2 | b | b | ND |
| NIH3T3 | Murine fibroblast cell line | 1.0 | 0.92 | b | b | ND |
| M1 | SV40 transformed cell line | 1.0 | 2.2 | b | b | ND |
| Hela | Human cervical carcinoma line | 1.0 | 2.16 | b | b | 1.6 |
| NC37 | EBV immortalized human B lymphocyte line | 1.0 | 4.5 | b | b | ND |
| HUT78 | Transformed human lymphocyte line | 1.0 | 4.5 | b | b | 2.1 |
| C8166 | HTLV-I infected non-producer | 1.0 | 74 | b | b | ND |
| HUT102 | HTLV-I producer T lymphocyte line | 1.0 | 28 | 1.4 | 1.7 | ND |
| MT2 | HTLV-I producer T lymphocyte line | 1.0 | 180 | ND | 5.5 | 2.4 |
| C344 | HTLV-II producer | 1.0 | 140 | 40 | 45 | ND |
| HOS | Human osteocarcinoma cell line | 1.0 | 0.7 | b | b | ND |
| HOS/M | HTLV-I infected | 1.0 | 75 | b | b | 1.0 |

| | | | | | | |
|---|---|---|---|---|---|---|
| a) The values shown represent a kinetic analysis of CAT activity directed by the individual plasmids relative to pSV2CAT; | | | | | | |
| b) CAT activity was too low to quantitate; | | | | | | |
| c) MD - No Data | | | | | | |

The level of CAT activity upon transfection of the plasmids that contain the HTLV-I LTR sequence into human fibroblast and epithelioid cells, HeLa (see, G.O. Gey et al., Cancer Res., 12: 264 (1952)), and Ml (see, B. Royer-Pokora et al., Exp. Cell Res., 151: 408 (1984)) was greater than that observed upon transfection of the same cells with the pSV2CAT containing plasmid. Negligible CAT activity was observed upon transfection of these cells with plasmids that contained HTLV Type II sequences. Thus, HTLV-I LTR sequences can function as transcriptional control elements in human cells that are not the natural targets for HTLV-Infection, whereas the HTLV-II LTR sequences cannot.

LTR-mediated retroviral gene expression in expression cells occurs from a provirus stably integrated into the host cell genome, whereas in a transient assay the transfected DNA directing transcriptional exists primarily in an extrachromosomal state (see, B. Howard et al., Biochem. Biophys. Acta, 228: 105 (1971); and A. Loyter et al., Proc. Natl. Acad. Sci. U.S.A., 79: 422 (1982).

To determine if the results obtained from transient assays were relevant to an understanding of HTLV LTR function in the integrated provirus, the HTLV LTR sequences used to direct CAT expression were positioned upstream of the neomycin phosphotransferase (NEO) gene (P. J. Southern et al., Mol. Appl. Gen., 1: 227 (1982)). These plasmids were transfected into murine and human cell lines (Table 5) and stably transfected colonies were selected in the presence of a neomycin analog G-418. The number of G-418 resistant colonies obtained relative to that of the SV40-promoted neomycin phosphotransferase gene demonstrated a close parallel in the transient expression data. The levels of CAT activity in transient assays provided reasonable insight into the ability of the transcriptional element being tested to function upon integration into the host chromosome.

**TABLE 5**

| Cell line | Colonies per dish | | | |
|---|---|---|---|---|
| | pU3R-I | pU3-II | PSV2NEO | RSVNEO |
| NIH 3T3 | 24 | 0 | 24 | 82 |
| HeLa | 15 | 0 | 7 | 97 |

The recombinant plasmids indicated in Table 5 were constructed by cleavage of the HTLV-CAT recombinants with Hind III-Bam HI and replacing the CAT coding sequence with the gene coding neomycin resistance. Twenty four hours prior to transfection 1 X 10⁶ cells were seeded into 100 cm² dishes. Transfection was done using the calcium phosphate 04-DNA coprecipitation method using 3 ug of CsCl banded DNA. After 48 hours cells were replated onto 150 cm² dishes and selected in medium containing G-418 (400 ug/ml). Colonies were counted 2-3 weeks after transfection.

### EXAMPLE 3

### Expression in Human Lymphoid Cells

In naturally acquired HTLV-Infection, the majority of infected cells are of the T cell lineage (see, R.C. Gallo et al., Proc. Natl. Acad. Sci. U.S.A., 79: 5680 (1982). However, some HTLV producing cells expressing B cell markers have been isolated (see for example, N. Yamamoto et al., Nature (London), 299: 367 (1982)). To determine the relative promotional strength of the LTR sequences within lymphocytes, cell lines of lymphoid origin were transfected with the plasmids described above. The cell lines used were: HUT78, an OKT4+ human T cell line derived from an HTLV-negative patient who with Sezary syndrome (this cell line lacks HTLV proviral sequences - for a description and original literature references for this cell line, see V. Manzari et al., Proc. Natl. Acad. Sci. U.S.A., 80: 11 (1983)); NC37, an Epstein-Barr virus - immortalized B cell line established from a normal donor (ibid); and L691, a murine lymphoid cell line that expresses T cell markers.

The LTR sequences of HTLV-I directed the synthesis of appreciable levels of the CAT gene product to that of pSV2CAT activity was approximately twice as high as this ratio in fibroblasts and epithelioid cell lines. Higher CAT activity relative to the level induced by pSV2CAT was observed in the murine cell line transfected with the plasmid pV3R-I. These findings demonstrate that both species-specific and cell type-specific factors modulate the activity of the HTLV-I LTR.

Surprisingly, transfection of the HUT78 or NC37 cells with plasmids that contained HTLV-II LTR sequences resulted in no appreciable level of CAT activity. This suggested that a human lymphoid environment is not sufficient for the efficient function of the HTLV-II LTR and that substantial differences in the requirements for the LTR function of HTLV-I and HTLV-II exist.

### EXAMPLE 4

### Expression in HTLV-Infected Cells

The inactivity of the HTLV-II LTR in the lymphoid cell lines suggested that factor specific to the HTLV target cell might be required for efficient expression of the CAT gene under control of the HTLV LTRs. For this reason HTLV-producing cell lines derived from infected individuals, or cell lines established by cocultivation of human primary lymphocytes were transfected with HTLV producer cell lines. The cell lines used included: HUT102, and HTLV-I producing OKT4+ cell line established from a patient with an HTLV associated adult T cell leukemia/lymphoma (mycosis fungocides - ibid); MT2, and HTLV-I producing OKT-4+ cell line established by immortalization of primary T lymphocytes after cocultivation with an HTLV producer cell line (see, I. Miyoshi et al., Nature (London), 294: 770 (1981)); C3-44, an immortalized HTLV-II producing cell line derived by cocultivation of primary lymphocytes with patient cells (Manzari, supra); and C81-66, an HTLV-I immortalized OKT4+ primary T cell that does not produce virus (see, B. Hanh et al., Nature (London), 303: 253 (1983)).

The results obtained upon transfection of these cell lines with the plasmid that contains the HTLV-I LTR sequences were remarkable and unexpected. The level of CAT activity was dramatically increased relative to that of pSV2CAT, rating from 25 to 180-fold greater.

Contributing to this large ratio of Type I LTR AT activity is a consistent decrease in the activity of the pSV2CAT and pSVCAT plasmids in these cells. This may be due to a decreased efficiency in the uptake and promotional activity of these elements. Normalizing expression of transfected DNA by these cells, or to specific or non-specific factors down-regulating the promotional activity of these elements. Normalizing for DNA uptake indicates that the increased level of CAT activity directed by the pU3-RI plasmid in infected cells represents a real and not solely a relative increase compared to levels in uninfected cells. Since the extrachromosomal state of transiently expressing DNA precludes efficient cis-activation, these results suggest that trans-acting factors in HTLV-infected cells stimulate the transcriptional ability of the HTLV LTR. Although low, the CAT activity directed by the plasmids containing HTLV-II LTR sequences is substantially higher in most of the cells that contain HTLV-I proviruses than it is in uninfected cells.

To determine if the HTLV-II LTR sequences could function in a cell line producing Type II virus, the same plasmids were used to transfect the C3-44 cell line. In this cell line, a very high level of CAT activity was observed for both the pU3R-II and pU3-II plasmids. The level of CAT activity is approximately forty times that of the same cells transfected with pSV2CAT DNA. This demonstrates that in the proper cellular environment, the HTLV-II LTR also functions as an efficient transcriptional element.

The CAT activity of the plasmid containing HTLV-I LTR sequences was also very high in C3-44 cells, approximately 135 times that of pSV2CAT. This, even in the target cell for HTLV-II infection, the HTLV-I LTR exhibits greater promotional strength than does the HTLV-II LTR.

T lymphocytes infected with HTLV exhibit changes that also characterize T cells activated by exposure to mitogenic or antigenic stimuli (M. Popovic, et al., Proc. Natl. Acad. Sci. U.S.A., 80: 5402 (1983)).

To examine whether T cell-activation alone might modify the cellular environment to allow increased transcriptional activity of the HTLV LTRs, the HTLV-CAT and control plasmids were transfected into an immature human T cell line, Jurkat, both in the presence and absence of the T cell mitogen phytohemaglutinin (PHA) (see, Manzari, supra). No effect of PHA stimulation on the relative levels of CAT activity was seen directed by any of the plasmids. It was concluded that PHA-activation of T cells alone was insufficient to account for the stimulation of CAT activity seen in infected cells transfected with the HTLV-CAT plasmids.

### EXAMPLE 5

### Test for Viral Associated Trans-acting Factors

To directly test whether the high degree of HTLV LTR function in HTLV-infected T lymphocyte subsets viral-encoded or -induced factors play a role in this phenomenon, the plasmids were introduced into HOS/M cells, a cell line established by infecting a human osteosarcoma line (HOS) in vitro with HTLV-I. Although HOS/M cells produce HTLV-I virions and express viral proteins, they do not express receptors for T cell growth factor, OKT antigens, new HLA antigens or lymphokines; properties that characterize HTLV-infected lymphocytes (see, P. Clapham et al., Science, 222: 1125 (1983)). Thus, although viral proteins are produced, the HOS/M cellular environment differs markedly from HTLV-infected tumor cells and should help distinguish between the above alternatives.

As noted supra, the HTLV-I LTR functioned in the uninfected HOS cells somewhat less efficiently than the SV40 early promoter. By contrast, PV3R-I directed CAT expression was fifty-fold higher than that of pSV2CAT in HOS-M cells. Moreover, consistent with our results in HTLV-I-infected lymphocytes, PVRR-II and PV3R-II do not express significant levels of CAT in the transfected HOS/M cells.

### EXAMPLE 6

### Deletion analysis

(a) For clarity the sequences present within the HTLV-I LTR U3 region of strain HTLV-CR are shown in Figure 1. The 51 bp imperfect repeats are overlined and the 21 bp imperfect repeats are underlined. Arrows indicate the position of each deletion described in part b, infra.
(b) To construct the deletion DNAs plasmid pU3R-I⁷ was first cleaved at the unique site Sma I site (position -322) within the LTR. Digested DNA was incubated with the exonuclease Bal 31. At various time points aliquots were removed and the DNA was phenol chloroform extracted then ethanol precipitated. Any protruding ends were filled with T4 polymerase followed by ligation to Xho I linkers. After linker addition the DNA was recircularized and transfected into E. coli. strain HB101. Several size selected clones were characterized by restriction enzyme analysis. The desired clones were then end labeled at the Xho I site and subjected to Maxam and Gilbert sequence analysis (see, Maxam, A. M. et al., Proc. Natl. Acad. Sci. USA, 74: 560-564 (1977)) to determine the end points. The name of each plasmid is indicative of the number of nucleotides present relative to the transcription start site.
(c) The deletion DNAs were transfected into the following cell types by a modification of the DEAE dextran coprecipitation technique (see, Queen, C. et al., Cell, 33: 729 (1983)). Hela, a human epithelial cell line; HUT 78, an OKT4+ helper/inducer human T cell line derived from an HTLV negative patient with sezary syndrome (see, Gey, et al., Cancer Res., 12: 264 (1952)); NC37, a B lymphocyte line established from a normal donor and immortalized by Epstein Barr virus (see Manzari, V. et al., Proc. Natl. Acad. Sci. USA, 80: 11-15 (1983)); and 681-66/45, derived by fusion of primary umbilical cord blood cells with an HTLV-I producing cell line (see Salahuddin, S. Z. et al., Virology, 129: 51-64 (1983)). This cell line does not produce virus but contains the HTLV-I associated trans-acting factor. Forty eight hours following transfection cellular extracts were trade and CAT assays were performed as previously described. The values shown are normalized to the CAT activity present in similar cells transfected with the parental plasmid pU3R-I taken as 1.0. The bracketed number indicates the relative CAT activity in C81-66/45 cells normalized to the activity present in the uninfected lymphocyte lines transfected with the pSV2CAT plasmid that contains the SV40 enhancer promoter sequences (Laimins et al., supra).

### EXAMPLE 7

### Construction of Recombinant Plasmids

The upper circle denotes the vector plasmid pSVIXCAT. Plasmid pSVIXCAT lacks most of the 72 bp repeat enhancer sequences, but retains the SV40 early promoter region positioned 5' to the CAT gene. The plasmids pHE1CAT, pHE2CAT and pHE3CAT containing the HTLV U3 sequences positioned 5' to the SV40 early promoter were constructed as follows: plasmid pU3R-I was cleaved with Nde I which cuts once in the pBR322 sequences and 35 bp 5' to the HTLV TATA sequences. The 450 bp fragment was gel isolated and ligated to Nde I cleaved PSVIXCAT. DNA was transfected into HB101 and colonies were scored for the presence and orientation of HTLV inserts. Plasmid pHX1CAT which contains the same HTLV U3 sequence but positioned in the reverse orientation on the HTLV promoter was constructed by cleaving pU3R-I with Nde I, religating the DNA, and screening the reclosures for reverse orientation inserts. Plasmids pREHl and pRESl contain the LTR enhancer sequences respectively. Plasmid pRESl was constructed by cleaving PSV2CAT with AccI- SpHI to delete the SV40 enhancer sequences. The protruding ends were filled with T4 polymerase and the blunt ended DNA was ligated to Xho l linkers. DNA was then cleaved with Xhol-BamHl and the fragment containing the SV40 early promoter region and the CAT gene was ligated to an Xhol-BamHl cleaved variant of RSVCAT. Thus, positioning the RSV enhancers 5' to the SV40 early promoter. To construct pREHl the Nde l site of pU3R-I was converted to an Xho I site. Following digestion with Xho I-HindIII the fragment containing the HTLV promoter sequences was used to replace the Xho I-HindIII fragment of pRESI. All recombinant DNA techniques were conducted according to standard procedures (see, Maniatis, T., Fritch, E. F., and Sambrook, J., "Molecular Cloning" (Cold Springs Harbor Laboratory, 1982)). Enzyme digestions were conducted according to the manufacturers specifications. The blackened and crosshatched rectangular bars represent HTLV and RSV sequences respectively. Arrow directions indicate the orientation of sequences with respect to their orientation in the LTR. Blackened and open squares represent HTLV and SV40 promoter sequences respectively. Sequences derived from plasmid pBR322 are shown as solid lines.

### EXAMPLE 8

### Relative CAT Activity Directed by the HTLV U3 Sequence

The recombinant plasmids bearing the HTLV U3 sequences 5' to the SV40 early promoter region were transfected into Hela cells by the CaPO₄ co-precipitation method (see, Graham, F. and van der Eb, A., Virology, 52: 456-467 (1973)). After a 48 hour incubation cellular extracts were prepared. Cell extracts (200-400 ug of protein) were incubated with 140 chloramphenicol and Acetyl CoA (24 mM). A time course assay was done and acetylated forms of chloramphenicol were separated from the non-acetylated substrate by ascending thin layer chromatography (see, Gorman et al., Molec. Cell. Biol., supra). The CAT gene expression directed by the constructs are expressed relative to the CAT activity present in cells transfected with pSVIXCAT taken as 1.0. The results represent an average of at least two separate experiments. Insets depict autoradiograms of typical CAT assays.

### EXAMPLE 9

The recipient vector contains a Hind III-XhoI fragment of plasmid pSVIXCAT, a variant of plasmid pSVICAT. pSV2CAT contains the SV40 early promoter enhancer region located 5' to the gene which codes for production of chloramphenicol acetyl transferase (CAT). PSVIXCAT is identical to PSV2CAT except it lacks the 72 bp enhancer region. Digestion with Hind III-XhoI removes the SV40 promoter region, then allowing HTLV sequences to be placed 5' to CAT. The following plasmids were constructed: PV3R-I; a proviral clone containing the 3' LTR or HTLV-I was cleaved with RSAI and ligated to XhoI linker, DNA was then cleaved with Mbol, filled out with T4 polymerase and ligated to Hind III linkers. After Hind III-XhoI cleaving the 800 bp fragment was ligated to the CAT vector. PV3-II, and HTLV-II proviral clone containing a 5' LTR was cleaved with EcoRi, filled in with T4 polymerase, ligated to Hind III linker, cleaved with Hind III-XhoI then the bp fragment was ligated to the CAT Vector. PV3-RII; and HTLV-II proviral clone containing a 3' LTR was cleaved with BAMHI, filled out with T4 polymerase, and ligated to HIND III linkers. Digestion with HIND III-XhoI produced a fragment that was ligated to the CAT vector. All constructions were confirmed by restriction enzyme mapping. Plasmid DNAs were purified by centrifugation in cesium chloride gradients.

### EXAMPLE 10

NIH3T3 and CV-l cells were transfected by a modification of the calcium phosphate coprecipitation technique (Graham, supra). Approximately 1 X 10⁶ cells were seeded on 10 cm² dishes 24 hours prior to addition of the Capo4-DNA precipitate. One ml of the precipitate containing 5-10 ug of DNA was added to the medium, and the cells were incubated 4 hours at 37°C, then subjected to a 3 minute glycerol shock. CV-l cells were subjected to a 10 minute DMSO shock 24 hours after transfection. All other cell lines were transfected by a modification of the DEAE-dextran technique (Queen et al., supra). Twenty-four hours before transfection adherent cells were seeded at a density of 10⁶ cells per 10 cm² dish. Immediately prior to transfection cells were typsinized, washed, and transfected in suspension with 5-8 ug of DNA an amount shown to be below saturation. Lymphocyte lines were transfected at a density of 1-5 X 10⁶ cells per ml using 5-10 ug of DNA. Dot blot analysis confirmed comparable uptake between all cell lines. Cells were harvested 48 hours after transfection, and cellular extracts were prepared by freeze/thawing (three times). After a brief centrifugation to remove cell debris, extracts were analyzed for CAT activity as described by Gorman except that acetyl coenzyme A was present at 24 mm. Percent conversion of chloramphenicol to the acetylated forms was determined by ascending thin layer chromatography and liquid scintillation counting of the spots cut from the plate.

### EXAMPLE 11

### Establishment of Stable Cell Lines Producing the HTLV-I tat Transcription Factor.

The eukaryotic selectable marker, pSV2gpt, was inserted into the CATLOR_{II} plasmid. This DNA was transfected into NIH3T3 murine fibroblasts, CCCS+L-feline kidney cells and HeLa human cervical carcinoma cells by calcium phosphate precipitation. 48 hours after transfection, cells were placed into xanthine:mycophenolic acid - containing medium, which selects colonies of cells that have stably integrated the CATLOR_{II}-pSV2gpt plasmid into their chromosomes. Clones of these cells were isolated and tested for the presence of trans-acting factors specifically activating transcription directed by the HTLV LTR.

Several clones were isolated that express the tat protein and no other HTLV viral proteins. The HTLV LTR exhibits a dramatic increase in promoter activity in these cell lines, compared to the parental control cell lines. Thus any gene under the control of the HTLV LTR can be expressed at very high levels (about 30 times the level obtained with the SV40 early promoter) in these cloned cell lines.

The HTLV LTR sequences of this invention are useful for the overproduction of proteins and like materials expressable by genes. In order to cause this overproduction, an HTLV LTR is first linked to the particular heterologous gene of interest in a configuration suitable for use of the HTLV LTR as a promoter region, and then that construct is employed in the transfection of a cell containing an HTLV genome which functions in a trans-acting manner. If desired, the entire region of the HTLV LTR need not be utilized. For example, for HTLV-I, the region from -159 to +315 is sufficient for the response to trans-acting factors. It is capable of being complemented with its own or heterologous enhancer elements to make a trans-activated promoter element. Thus, the concept utilizes a heterologous construction for the overproduction of expressable material.

A second embodiment for the overproduction of a protein or like product involves constructing a vector containing the tat sequence, the HTLV LTR sequence and the heterologous gene. The heterologous gene must be configured so as to permit splicing and expression. The cell, now transfected with this material, is driven to super activity levels by tat and HTLV LTR, resulting in the overproduction of the heterologous gene product.

As employed herein, the generic term HTLV refers to all three viruses, type I, II and III. Regardless of which HTLV-Is employed, the heterologous gene product will be greatly stimulated in the presence of the appropriate tat gene product and the HTLV LTR.

Another utility envisioned for the HTLV LTR vectors of this invention makes use of the fact that when you hook the HTLV LTR onto a gene that is either linked to a cell or which expresses a surface protein, you permit recognition of that cell and consequent destruction thereof by the use of monoclonal or polyclonal antibodies. This is especially important in the treatment of HTLV mediated diseses, including AIDS.

Finally, it should be noted that the HTLV LTR vectors of the present invention may be employed in the production of vaccines. The first type of vaccine employs empty capsids (Mann et al., supra) which can be produced by the deletion of a region which is non-essential for virion capsid production. These plasmids can be employed to transfect a cell known to express virion proteins, such as for example HOS or HeLa. Here, the LTR would function, even in the absence of the trans-acting factor (tat, formerly called luk)) to express virions at a low level. This low virion level induces immunity, yet doesn't induce the viral disease.

Vaccine production involves the infection of a cell which contains at a different chromosomal site, the tat gene, preengineered for expression, but not for expression of other viral proteins. A deletion in a pro-virus is first inserted into a cell containing twice the LTR's of HTLV, plus the tat region, but containing no other viral RNA. This construct then trans-activates a second virus transfected into the cell and thus produce a high rate of viriality. Any of the HTLV LTR elements can be used in this construct. For example, if it is discovered that the HTLV-III LTR is itself deliterious, the LTR for either HTLV-I or HTLV-II can be switched therefor.

In order to create a live attenuated vaccine, the functional part of the tat region is deleted, thus limiting the trans-activating ability of the virion. The virus is still be capable of infecting cells, but is no longer capable of overproduction and therefore causing disease.

A further extention or utility for the HTLV LTR's of the present invention, on portions thereof (especially the tissue specific determinants) represents the first pharmaceutical use of these species. For example, the HTLV LTR's may be combined with an appropriate pharmaceutical carrier for delivery to a mammal of the tissue specific entity. Moreover, the tissue specific vectors may be employed in the delivery of antitumor on like pharmaceutical agents, such as those described in the Physicians' Desk Reference, 38th ed., Medical Economics Co., Oraden, N.J. (1984), the disclosure of which is incorporated herein by reference.

## Claims

1. A gene expression system comprising:
(a) an expression vector comprising
(i) a cis-acting regulatory element corresponding to a sufficient portion of a region in the U3 and/or R regions of an HTLV or HIV LTR to be responsive to a trans-acting protein, wherein said trans-acting protein is expressed by a trans-acting DNA segment, wherein said trans-acting DNA segment corresponds to a sufficient portion of (1) a long open reading frame (LOR) which is 3' to the envelope gene in HTLV-I, HTLV-2 and BLV and has a consensus splice acceptor site at the 5' end, and a start codon in the proper reading frame therewith capable of encoding a trans-acting protein which has a molecular weight of about 38-42,000 or (2) an exon between the HIV pol and env genes in an alternate reading frame therefrom to encode a trans-acting protein having a proline rich region, a cysteine rich region and a basic region rich in arginine and lysine,
(ii) and a heterologous gene under the control of the cis-acting regulatory element; and
(b) the trans-acting DNA segment operably-linked to a sequence which will permit expression of the trans-acting protein,
wherein expression of said trans-acting protein is capable of enhancing expression of the heterologous gene.

2. The gene expression system of claim 1, wherein said trans-acting DNA segment comprises the tat gene, and said tat gene of HTLV corresponds to an LOR region in the 3' protein of an HTLV-I, HTLV-II or BLV viral genome, 3' to the env gene, with a consensus splice acceptor site at the 5' end of the LOR and a start codon in the proper reading frame therewith, and said tat gene of HIV corresponds to a first exon in an HIV viral genome between the env gene and the pol gene, capable of encoding a trans-acting protein having a proline-rich region, a cysteine rich region and a basic region rich in arginine and lysine.

3. The gene expression system of claim 2, wherein the tat gene is an HIV tat gene.

4. The gene expression system of claim 2, wherein the tat gene is an HTLV-I, HTLV-II or BLV tat gene.

5. The gene expression system of claim 4, wherein the start codon is the initiation codon at the 5' end of the env gene.

6. The gene expression system of claim 4, wherein a start codon is fused in frame with the LOR reading frame.

7. The gene expression system of claim 1, wherein the cis-acting regulatory element responsive to said trans-acting protein is referred to as the tar element.

8. The gene expression system of claim 7, wherein the cis-acting regulatory element comprises the tar element of HTLV-I, HTLV-II, BLV or HIV.

9. The gene expression system of claim 1, wherein the cis-acting regulatory element corresponds to an HIV viral genome and contains a region in the U3 region located between about HIV nucleotides -340 to -185 that is capable of having a negative regulatory effect on genes operably linked to the HIV LTR, referred to herein as an NRE region, and also contains a region in the R region of the HIV LTR that is responsive to the trans-acting protein referred to as the tar element.

10. The gene expression system of claim 9, wherein the tar element is located between about HIV nucleotides -17 to +80.

11. The gene expression system of claim 1, wherein the cis-acting regulatory element corresponds to a region in an HIV viral genome in the R region of the LTR and is referred to as the tar element.

12. A method for stimulating the production of a gene product, which comprises:
a) inserting a trans-acting DNA segment into a host cell, wherein said trans-acting DNA segment corresponds to a sufficient portion of (1) a long open reading frame (LOR) which is 3' to the env gene in HTLV-I, HTLV-2 and BLV and has a consensus splice acceptor site at the 5' end, and a start codon in the proper reading frame therewith capable of encoding a trans-acting protein which has a molecular weight of about 38-42,000 or (2) an exon between the HIV pol and env genes in an alternate reading frame therefrom to encode a trans-acting protein having a proline rich region, a cysteine rich region and a basic region rich in arginine and lysine,
b) inserting an expression vector into the host cell, wherein the expression vector comprises a cis-acting regulatory element corresponding to a sufficient portion of a region from the U3 and/or R region of an HTLV- or HIV LTR to be responsive to the trans-acting protein expressed by the trans-acting DNA segment, and a gene under the control of the cis-acting regulatory element; and
(c) cultivating the host cell.

13. The method of claim 12, wherein the gene to be expressed is heterologous to the genome of the trans-acting protein.

14. The method of claim 12, wherein the trans-acting DNA segment comprises the tat gene, and said tat gene of HTLV corresponds to an LOR region in the 3' portion of an HTLV-I, HTLV-II or BLV viral genome, 3' to the env gene, with a consensus splice acceptor site at the 5' end of the LOR with the start codon in the proper reading frame therewith, and said tat gene of HIV corresponds to a first exon in an HIV viral genome between the env gene and the pol gene, capable of encoding a trans-acting protein having a proline-rich region, a cysteine rich region and a basic region rich in arginine and lysine.

15. The method of claim 14, wherein the tat gene is an HTLV-I, HTLV-II, BLV or HIV tat gene.

16. The method of claim 14, wherein the tat gene is an HIV tat gene.

17. The method of claim 12, wherein the cis-acting regulatory element responsive to said trans-acting protein is referred to as the tar element.

18. The method of claim 17, wherein the cis-acting regulatory element comprises the tar element of HTLV-I, HTLV-II, BLV or HIV.

19. The method of claim 12, wherein the cis-acting regulatory element corresponds to a region in an HIV viral genome in the R region of the LTR and is referred to as the tar element.

20. The method claim 19, wherein the tar element is located between about HIV nucleotides -17 to +80.

21. The method of claim 12, wherein the cis-acting regulatory element corresponds to an HIV viral genome and contains a region in the U3 region located between about HIV nucleotides -340 to -185 having a negative regulatory effect on genes operably linked to the HIV LTR, referred to herein as an NRE region, and also contains a region in the R region of the HIV LTR that is responsive to the trans -acting protein referred to as the tar element.

22. The method of claim 12, wherein the level of production of the gene product in the presence of the trans-acting factor is at least five times greater that the level in the absence of the trans-acting factor.

23. The method of claim 12, wherein the host cell is a fibroblast cell, a lymphoid cell, an epithelial cell, or a hamster ovary cell.

24. The method of claim 23, wherein the host cell is of human, murine, feline or simian origin.

25. The method of claim 24, wherein the host cell is a fibroblast, lymphocyte or epithelial cell

26. A DNA segment comprising a trans-acting DNA segment wherein said trans-acting DNA segment corresponds to a sufficient portion of (1) a long open reading frame (LOR) which is 3' to the envelope gene in HTLV-I, HTLV-2 and BLV and has a consensus splice acceptor site at the 5' end, and a start codon in the proper reading frame therewith capable of encoding a trans-acting protein which has a molecular weight of about 38-42,000 or (2) an exon between the HIV pol and env genes in an alternate reading frame therefrom to encode a trans-acting protein having a proline rich region, a cysteine rich region and a basic region rich in arginine and lysine
wherein expression of said trans-acting protein is capable of enhancing expression of a heterologous gene operably linked to a 5' LTR of the same genome.

## Patentansprüche

1. Genexpressionssystem, umfassend:
a) einen Expressionsvektor, umfassend
(i) ein cis-wirkendes regulatorisches Element, das einem ausreichenden Abschnitt eines Bereiches in dem U3- und/oder R-Bereich einer HTLV oder HIV LTR entspricht, um auf ein trans-wirkendes Protein anzusprechen, wobei das trans-wirkende Protein durch ein trans-wirkendes DNA-Segment exprimiert wird, wobei das trans-wirkende DNA-Segment einem ausreichenden Abschnitt eines (1) langen offenen Leserasters (LOR) entspricht, das 3' zum Hüllgen in HTLV-I, HTLV-2 und BLV ist und eine Konsensus-Spleißakzeptorstelle am 5'-Ende sowie ein Startcodon in dem mit diesem passenden Leseraster aufweist und in der Lage ist, für ein trans-wirkendes Protein zu codieren, das ein Molekulargewicht von etwa 38000 bis 42000 hat, oder (2) eines Exons zwischen dem HIV pol- und env-Gen in einem von diesem versetzten Leseraster, um für ein trans-wirkendes Protein mit einem prolinreichen Bereich, einem cysteinreichen Bereich und einem an Arginin und Lysin reichen Basisbereich zu codieren,
(ii) und ein heterologes Gen unter der Kontrolle des cis-wirkenden regulatorischen Elements; und
(b) das mit einer Sequenz wirkverbundene trans-wirkende DNA-Segment, die die Expression des trans-wirkenden Proteins erlaubt,
wobei die Expression des trans-wirkenden Proteins in der Lage ist, die Expression des heterologen Gens zu verbessern.

2. Genexpressionssystem nach Anspruch 1, wobei das trans-wirkenden DNA-Segment das tat-Gen umfaßt und das HTLV-tat-Gen einem LOR-Bereich im 3'-Protein eines HTLV-I, HTLV-II oder BLV-viralen Genoms entspricht, 3' zum env-Gen, mit einer Konsensus-Spleißakzeptorstelle am 5'-Ende des LOR und einem Startcodon in dem mit diesem passenden Leseraster, und das HIV-tat-Gen einem ersten Exon in einem HIV-viralen Genom zwischen dem env-Gen und dem pol-Gen entspricht, das in der Lage ist, für ein trans-wirkendes Protein mit einem prolinreichen Bereich, einem cysteinreichen Bereich und einem an Arginin und Lysin reichen Basisbereich zu codieren.

3. Genexpressionssystem nach Anspruch 2, wobei das tat-Gen ein HIV tat-Gen ist.

4. Genexpressionssystem nach Anspruch 2, wobei das tat-Gen ein HTLV-I, HTLV-II oder BLV tat-Gen ist.

5. Genexpressionssystem nach Anspruch 4, wobei das Startcodon das Initiationscodon am 5'-Ende des env-Gens ist.

6. Genexpressionssystem nach Anspruch 4, wobei ein Startcodon im Raster mit dem LOR-Leseraster verschmolzen ist.

7. Genexpressionssystem nach Anspruch 1, wobei das auf das trans-wirkenden Protein ansprechende cis-wirkende regulatorische Element als das tar-Element bezeichnet wird.

8. Genexpressionssystem nach Anspruch 7, wobei das cis-wirkende regulatorische Element das tar-Element von HTLV-I, HTLV-II, BLV oder HIV enthält.

9. Genexpressionssystem nach Anspruch 1, wobei das cis-wirkende regulatorische Element einem HIV-viralen Genom entspricht und einen Bereich in dem U3-Bereich enthält, der etwa zwischen den HIV-Nukleotiden -340 bis -185 angeordnet ist, der in der Lage ist, einen negativen regulatorischen Effekt auf mit der HIV-LTR wirkverbundene Gene auszuüben, hierin als NRE-Bereich bezeichnet, und ferner einen Bereich im R-Bereich der HIV-LTR enthält, der auf das als das tar-Element bezeichnete trans-wirkende Protein anspricht.

10. Genexpressionssystem nach Anspruch 9, wobei das tar-Element etwa zwischen den HIV-Nukleotiden -17 bis +80 angeordnet ist.

11. Genexpressionssystem nach Anspruch 1, wobei das cis-wirkende regulatorische Element einem Bereich in einem HIV-viralen Genom in dem R-Bereich der LTR entspricht und als tar-Element bezeichnet wird.

12. Verfahren zur Stimulierung der Produktion eines Genprodukts, umfassend:
a) Einsetzen eines trans-wirkenden DNA-Segments in eine Wirtszelle, wobei das trans-wirkende DNA-Segment einem ausreichenden Abschnitt (1) eines langen offenen Leserasters (LOR) entspricht, das 3' zum env-Gen in HTLV-I, HTLV-2 und BLV ist und eine Konsensus-Spleißakzeptorstelle am 5'-Ende sowie ein Startcodon in dem mit diesem passenden Leseraster aufweist, das in der Lage ist, für ein trans-wirkendes Protein zu codieren, das ein Molekulargewicht von etwa 38000 bis 42000 hat, oder (2) eines Exons zwischen dem HIV pol- und env-Gen in einem von diesem versetzten Leseraster, um für ein trans-wirkendes Protein mit einem prolinreichen Bereich, einem cysteinreichen Bereich und einem an Arginin und Lysin reichen Basisbereich zu codieren,
b) Einsetzen eines Expressionsvektors in die Wirtszelle, wobei der Expressionsvektor ein cis-wirkendes regulatorisches Element umfaßt, das einem ausreichenden Abschnitt eines Bereiches aus dem U3- und/oder R-Bereich einer HTLV- oder HIV-LTR entspricht, um auf das von dem trans-wirkenden DNA-Segment exprimierte trans-wirkende Protein zu reagieren, sowie ein Gen unter der Kontrolle des cis-wirkenden regulatorischen Elements; und
c) Kultivieren der Wirtszelle.

13. Verfahren nach Anspruch 12, wobei das zu exprimierende Gen zum Genom des trans-wirkenden Proteins heterolog ist.

14. Verfahren nach Anspruch 12, wobei das trans-wirkende DNA-Segment das tat-Gen umfaßt und das HTLV-tat-Gen einem LOR-Bereich im 3'-Abschnitt eines HTLV-I, HTLV-II oder BLV-viralen Genoms entspricht, 3' zum env-Gen, mit einer Konsensus-Spleißakzeptorstelle am 5'-Ende des LOR mit dem Startcodon im mit diesem passenden Leseraster, und das HIV-tat-Gen einem ersten Exon in einem HIV-viralen Genom zwischen dem env-Gen und dem pol-Gen entspricht, das in der Lage ist, für ein trans-wirkendes Protein mit einem prolinreichen Bereich, einem cysteinreichen Bereich und einem an Arginin und Lysin reichen Basisbereich zu codieren.

15. Verfahren nach Anspruch 14, wobei das tat-Gen ein HTLV-I, HTLV-II, BLV oder HIV tat-Gen ist.

16. Verfahren nach Anspruch 14, wobei das tat-Gen ein HIV tat-Gen ist.

17. Verfahren nach Anspruch 12, wobei das auf das trans-wirkende Protein reagierende cis-wirkende regulatorische Element als das tar-Element bezeichnet wird.

18. Verfahren nach Anspruch 17, wobei das cis-wirkende regulatorische Element das tar-Element von HTLV-I, HTLV-II, BLV oder HIV umfaßt.

19. Verfahren nach Anspruch 12, wobei das cis-wirkende regulatorische Element einem Bereich in einem HIV-viralen Genom im R-Bereich der LTR entspricht und als das tar-Element bezeichnet wird.

20. Verfahren nach Anspruch 19, wobei das tar-Element etwa zwischen den HIV-Nukleotiden -17 bis +80 angeordnet ist.

21. Verfahren nach Anspruch 12, wobei das cis-wirkende regulatorische Element einem HIV-viralen Genom entspricht und einen Bereich im U3-Bereich enthält, der etwa zwischen HIV-Nukleotiden -340 bis -185 angeordnet ist und einen negativen regulatorischen Effekt auf mit der HIV LTR wirkverbundene Gene aufweist, hierin als NRE-Bereich bezeichnet, und ferner einen Bereich im R-Bereich der HIV LTR enthält, der auf das als das tar-Element bezeichnete trans-wirkende Protein anspricht.

22. Verfahren nach Anspruch 12, wobei der Produktionsspiegel des Genprodukts in Anwesenheit des trans-wirkenden Faktors wenigstens fünfmal größer als der Spiegel in Abwesenheit des trans-wirkenden Faktors ist.

23. Verfahren nach Anspruch 12, wobei die Wirtszelle eine Fibroblastenzelle, eine Lymphzelle, eine Epithelzelle oder Hamstereierstockzelle ist.

24. Verfahren nach Anspruch 23, wobei die Wirtszelle vom Menschen, von der Maus, von der Katze oder vom Affen stammt.

25. Verfahren nach Anspruch 24, wobei die Wirtszelle eine Fibroblaste, Lymphozyte oder Epithelzelle ist.

26. DNA-Segment, umfassend ein trans-wirkendes DNA-Segment, wobei das trans-wirkende DNA-Segment einem ausreichenden Abschnitt (1) eines langen offenen Leserasters (LOR) entspricht, der 3' zum Hüllgen in HTLV-I, HTLV-2 und BLV ist und eine Konsensus-Spleißakzeptorstelle am 5'-Ende und ein Startcodon im mit diesem passenden Leseraster aufweist, das in der Lage ist, für ein trans-wirkendes Protein zu codieren, das ein Molekulargewicht von etwa 38000 bis 42000 hat, oder (2) eines Exons zwischen dem HIV pol- und env-Gen in einem von diesem versetzten Leseraster, um für ein trans-wirkendes Protein mit einem prolinreichen Bereich, einem cysteinreichen Bereich und einem an Arginin und Lysin reichen Bereich zu codieren, wobei die Expression des trans-wirkenden Proteins in der Lage ist, die Expression eines mit einer 5'-LTR desselben Genoms wirkverbundenen heterologen Gens zu verbessern.

## Revendications

1. Système d'expression de gène, comprenant:
(a) un vecteur d'expression comprenant
(i) un élément de régulation à action cis correspondant à une portion suffisante d'une région dans les régions U3 et/ou R d'une longue répétition terminale (LTR) de HTLV ou de HIV pour être sensible à une protéine à action trans, ladite protéine à action trans étant exprimée par un segment d'ADN à action trans, ledit segment d'ADN à action trans correspondant à une portion suffisante (1) d'un long cadre de lecture ouvert (LOR) qui est en position 3' par rapport au gène d'enveloppe dans le HTLV-I, le HTLV-2 et le BLV et qui possède un site accepteur d'épissage consensus à l'extrémité 5', et un codon d'initiation dans le cadre de lecture convenable avec celui-ci, capable de coder pour une protéine à action trans qui possède un poids moléculaire d'environ 38-42 000 ou (2) d'un exon entre les gènes pol et env de HIV dans un autre cadre de lecture pour coder une protéine a action trans possédant une région riche en proline, une région riche en cystéine et une région basique riche en arginine et en lysine, et
(ii) un gêne hétérologue sous le contrôle de l'élément de régulation à action cis ; et
(b) le segment d'ADN à action trans lié de manière fonctionnelle à une séquence qui permet l'expression de la protéine à action trans,
dans lequel l'expression de ladite protéine à action trans est capable d'amplifier l'expression du gène hétérologue.

2. Système d'expression de gène suivant la revendication 1, dans lequel le segment d'ADN à action trans comprend le gène tat, et ledit gêne tat du HTLV correspond à une région LOR dans la protéine 3' d'un génome viral de HTLV-I, de HTLV-II ou de BLV, en position 3' par rapport au gène env avec un site accepteur d'épissage consensus à l'extrémité 5' du LOR et un codon d'initialisation en cadre de lecture convenable avec celui-ci, et ledit gène tat de HIV correspond à un premier exon dans un génome viral de HIV entre le gène env et le gène pol, capable de coder pour une protéine à action trans possédant une région riche en protéine, une région riche en cystéine et une région basique riche en arginine ou en lysine.

3. Système d'expression de gène suivant la revendication 2, dans lequel le gène tat est un gène tat de HIV.

4. Système d'expression de gène suivant la revendication 2, dans lequel le gène tat est un gène tat de HTLV-I, de HTLV-II ou de BLV.

5. Système d'expression de gène suivant la revendication 4, dans lequel le codon d'initiation est le codon d'initiation à l'extrémité 5' du gène env.

6. Système d'expression de gène suivant la revendication 4, dans lequel un codon d'initiation est fusionné en cadre avec le cadre de lecture LOR.

7. Système d'expression de gène suivant la revendication 1, dans lequel l'élément de régulation à action cis sensible à la protéine à action trans est désigné sous le nom d'élément tar.

8. Système d'expression de gène suivant la revendication 7, dans lequel l'élément de régulation à action cis comprend l'élément tar de HTLV-I, de HTLV-II, de BLV ou de HIV.

9. Système d'expression de gène suivant la revendication 1, dans lequel l'élément de régulation à action cis correspond à un génome viral de HIV et contient une région dans la région U3 située approximativement entre les nucléotides -340 et -185 du HIV qui est capable de posséder un effet de régulation négative sur les gènes liés de manière fonctionnelle à la LTR de HIV, désignée dans la présente invention sous le nom de région NRE, et contient également une région dans la région R de la LTR de HIV qui est sensible à la protéine action trans, désignée sous le nom d'élément tar.

10. Système d'expression de gène suivant la revendication 9, dans lequel l'élément tar est situé approximativement entre les nucléotides -17 et +80 du HIV.

11. Système d'expression de gène suivant la revendication 1, dans lequel l'élément de régulation à action cis correspond à une région dans un génome viral de HIV dans la région R de la LTR et est désigné sous le nom d'élément tar.

12. Procédé pour stimuler la formation du produit d'un gène, qui comprend :
a) l'insertion d'un segment d'ADN à action trans dans un cellule hôte, dans laquelle le segment d'ADN à action trans correspond à une portion suffisante (1) d'un long cadre de lecture ouvert (LOR) qui est en position 3' par rapport au gène env dans le HTLV-I, HTLV-II et le BLV et qui possède un site accepteur d'épissage consensus à l'extrémité 5', et un codon d'initiation en cadre de lecture convenable avec celuici, capable de coder pour une protéine à action trans qui possède un poids moléculaire d'environ 38-42 000, ou (2) d'un exon entre les gènes pol et env de HVI dans un autre cadre de lecture pour le codage pour une protéine à action trans possédant une région riche en proline, une région riche en cystéine et une région basique riche en arginine et en lysine,
b) l'insertion d'un vecteur d'expression dans la cellule hôte, dans laquelle le vecteur d'expression comprend un élément de régulation à action cis correspondant à une portion suffisante d'une région provenant de la région U3 et/ou R d'une LTR de HTLV ou de HIV pour être sensible à la protéine à action trans exprimée par le segment d'ADN à action trans et un gène sous le contrôle de l'élément de régulation à action cis ; et
c) la culture de la cellule hôte.

13. Procédé suivant la revendication 12, dans lequel le gène à exprimer est hétérologue vis-à-vis du génome de la protéine à action trans.

14. Procédé suivant la revendication 12, dans lequel le segment d'ADN à action trans comprend le gène tat, et ledit gène tat de HTLV correspond à une région LOR dans la portion 3' d'un génome viral de HTLV-I, de HTLV-II ou de BLV, en position 3' par rapport au gène env, avec un site accepteur d'épissage consensus à l'extrémité 5' du LOR avec le codon d'initiation en cadre de lecture convenable avec celuici, et ledit gène tat de HIV correspond à un premier exon dans un génome viral de HIV entre le gène env et le gène pol, capable de coder pour une protéine à action trans possédant une région riche en proline, une région riche en cystéine et une région basique riche en arginine et en lysine.

15. Procédé suivant la revendication 14, dans lequel le gène tat est un gène tat de HTLV-I, de HTLV-II, de BLV ou de HIV.

16. Procédé suivant la revendication 14, dans lequel le gène tat est un gène tat de HIV.

17. Procédé suivant la revendication 12, dans lequel l'élément de régulation à action cis sensible à la protéine à action trans est désigné sous le nom d'élément tar.

18. Procédé suivant la revendication 17, dans lequel l'élément de régulation à action cis comprend l'élément tar de HTLV-I, de HTLV-II, de BLV ou de HIV.

19. Procédé suivant la revendication 12, dans lequel l'élément de régulation à action cis correspond à une région dans un génome viral de HIV dans la région R de la LTR et est désigné sous le nom d'élément tar.

20. Procédé suivant la revendication 19, dans lequel l'élément tar est situé approximativement entre les nucléotides -17 et +80 du HIV.

21. Procédé suivant la revendication 12, dans lequel l'élément de régulation à action cis correspond à un génome viral de HIV et contient une région dans la région U3 située approximativement entre les nucléotides -340 et -185 du HIV possédant un effet régulateur négatif sur les gènes liés de manière fonctionnelle à la LTR de HIV, désignée dans la présente invention sous le nom de région NRE, et contient également une région dans la région R de la LTR de HIV qui est sensible à la protéine à action trans désignée sous le nom d'élément tar.

22. Procédé suivant la revendication 12, dans lequel le taux de production du produit de gène en présence du facteur à action trans est au moins cinq fois supérieur au taux en l'absence du facteur à action trans.

23. Procédé suivant la revendication 12, dans lequel la cellule hôte est un fibroblaste, une cellule lymphoïde, une cellule épithéliale ou une cellule d'ovaire de hamster.

24. Procédé suivant la revendication 23, dans lequel la cellule hôte est d'origine humaine, murine, féline ou simienne.

25. Procédé suivant la revendication 24, dans lequel la cellule hôte est un fibroblaste, un lymphocyte ou une cellule épithéliale.

26. Segment d'ADN comprenant un segment d'ADN à action trans, dans lequel ledit segment d'ADN à action trans correspond à une portion suffisante (1) d'un long cadre de lecture ouvert (LOR) qui est en position 3' par rapport au gène d'enveloppe dans le HTLV-I, le HTLV-II et le BLV et qui possède un site accepteur d'épissage consensus à l'extrémité 5', et un codon d'initiation dans le cadre de lecture convenable avec celui-ci, capable de coder pour une protéine à action trans qui possède un poids moléculaire d'environ 38-42 000, ou (2) d'un exon entre les gènes pol et env de HIV dans un autre cadre de lecture pour le codage pour une protéine à action trans possédant une région riche en proline, une région riche en cystéine et une région basique riche en arginine et en lysine, l'expression de ladite protéine à action trans étant capable d'amplifier l'expression d'un gène hétérologue lié de manière fonctionnelle à une LTR en 5' du même génome.
